(19) Europäisches Patentamt
European Patent Office
Office européen des brevets

(11) EP 2 358 814 B1

(12) EUROPEAN PATENT SPECIFICATION

(45) Date of publication and mention of the grant of the patent:
**29.07.2015 Bulletin 2015/31**

(21) Application number: **09833046.7**

(22) Date of filing: **15.12.2009**

(51) Int Cl.:
*C08K 5/00* (2006.01) *C08K 5/1545* (2006.01)
*C08L 67/04* (2006.01) *C08J 5/00* (2006.01)
*A61L 29/14* (2006.01) *A61L 15/42* (2006.01)

(86) International application number:
**PCT/IB2009/055771**

(87) International publication number:
**WO 2010/070588 (24.06.2010 Gazette 2010/25)**

(54) **PLASTICIZER FOR THERMOPLASTIC POLYMER MATERIALS**

WEICHMACHER FÜR THERMOPLASTISCHE POLYMERMATERIALIEN

PLASTIFIANT POUR MATÉRIAUX POLYMÈRES THERMOPLASTIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.12.2008 US 340409**
**10.12.2009 US 635256**

(43) Date of publication of application:
**24.08.2011 Bulletin 2011/34**

(73) Proprietor: **Avent, Inc.**
**Alpharetta, GA 30004 (US)**

(72) Inventors:
- **MACDONALD, John, Gavin**
  **Decatur**
  **Georgia 30033 (US)**
- **ELSHANI, Teuta**
  **Roswell**
  **Georgia 30075 (US)**
- **HRISTOV, Hristo, A.**
  **Roswell**
  **Georgia 30076 (US)**
- **SMITH, Molly, K.**
  **Atlanta**
  **Georgia 30317 (US)**
- **WEART, Ilona, F.**
  **Roswell**
  **Georgia 30068 (US)**

(74) Representative: **Hoffmann, Benjamin**
**Dehns**
**St. Bride's House**
**10 Salisbury Square**
**London EC4Y 8JD (GB)**

(56) References cited:
**EP-A1- 1 580 229** **US-A- 3 903 024**
**US-A- 5 504 184** **US-A1- 2001 001 690**
**US-A1- 2004 022 755** **US-A1- 2008 161 508**
**US-B1- 6 235 825**

- **DATABASE WPI Week 197636 Thomson Scientific, London, GB; AN 1976-68317X XP002728884, -& SU 491 629 A1 (HETEROORG CPDS AS USSR) 15 November 1975 (1975-11-15)**

EP 2 358 814 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF INVENTION

The present invention pertains to certain chemical additives that can be incorporated into thermoplastic polymeric materials to increase the relative plasticity of the polymeric material. In particular, the present invention relates to the plasticizing effect of xanthenes or xanthene-based molecular structures in semi-crystalline thermoplastic polymers, wherein the semi-crystalline polymers are polyalkylcarboxylic acids.

BACKGROUND

Molecular shape and the way molecules are arranged in a solid are important factors in determining the macroscopic physical properties of polymers. The relative degree of either brittleness or plasticity of a particular polymer material is dependent on the molecular structure, conformation and orientation of the polymer. The general concept of self-assembly enters into the organization of molecules on the micro and macroscopic scale as they aggregate into more ordered structures. Crystallization of regular solids is an example of the self-assembly process, as is the spatial organization of liquid crystals.

Conventional thermoplastic polymers, such as polypropylene or polylactic acid, tend to be relatively hard and rigid, sometimes even brittle. Manufacturers have over the years tried to develop or modify conventional thermoplastic materials to make them more pliable or "softer," but few have had success. This need for a new material composition or method to modify the thermoplastic polymeric materials to increase their relative amorphous content remains unsatisfied. The present invention provides a plasticizer composition to address this unfulfilled need.

SUMMARY OF THE INVENTION

The present invention pertains to xanthenes and xanthenes-based molecular structures in semi-crystalline thermoplastic polymer that can result in a highly amorphous thermoplastic material composition, wherein the semi-crystalline polymer is a polyalkylcarboxylic acid. The new composition yields a polymer material that is simultaneously softer or more pliable and stronger or tougher than the original or control starting polymer. This feature is a unique and unexpected finding of the composition which offers both benefits. The present invention pertains to, in part, a thermoplastic polymeric composition having a starting base semi-crystalline polymer with a minimal crystalline content of 14% to 25% by weight of the polymer, and a plasticizer compound with a xanthene-based molecular structure dispersed among the polymer molecules, in an amount up to 6000 ppm wherein the semi-crystalline polymer is a polyalkylcarboxylic acid. When solidified at ambient room temperature, the polymer composition has a crystalline phase and an amorphous phase in a ratio range of 0-15:85-100, respectively when compared to the starting polymer which has 14-87:13-86 respectively. The plasticizer compound with a xanthene-based molecular structure is dispersed within the amorphous phase. The resulting thermoplastic polymeric composition is essentially amorphous and has a relative level of crystallinity of 40-99% less than that of the starting or a control thermoplastic polymer that does not include the plasticizer compound with a xanthene-based molecular structure therein.

Incorporation of the plasticizer compound into the thermoplastic polymeric composition can increase the relative toughness of the base or underlying thermoplastic polymer by about at least 50%, and the relative tolerance for stretching and elongation by about at least 30%, and increase in the both the strain at break and stress at break by about at least 40% when the samples are analyzed in the temperature range 30-50°C. This would be additional benefit in the use of this composition in absorbent articles and medical devices in contact or inserted into the body. The semi-crystalline polymer is a member of the polyalkylcarboxylic acids, for example, polylactic acid. The semi-crystalline control polymer can contain a crystalline content of about 14 to about 87% crystalline phase, about 13% to about 86% amorphous state. The plasticizer compound with a xanthene-based molecular structure is present at about 1000 ppm to 6000ppm, and may include xanthene and halogenated or mixed-halogenated xanthenes.

In another aspect, the present invention relates to an article of manufacture comprising a thermoplastic polymer, the thermoplastic polymer having a semi-crystalline polymer matrix incorporating a plasticizer comprising a xanthene molecule or a compound with a xanthene-based molecular structure, said semi-crystalline polymer contains a crystalline content of 0% to 15% crystalline phase, 100% to 85% amorphous state, said xanthene molecule or compound with a xanthene-based molecular structure is present in said polymer matrix in an amount of 3000 ppm up to 5000 ppm; wherein said semi-crystalline polymer is a polyalkylcarboxylic acid; and wherein the crystalline content of the polyalkylcarboxylic acid is computed from the crystalline index:

$$X\% = \frac{I_c}{I_t} = \frac{\int i_c k^2 dk}{\int i_t k^2 dk} \quad (1)$$

where $I_c$ is the XRD intensity scattered from the crystalline portion of the material, $I_t$ is the total XRD intensity and k is the scattering vector with a magnitude $k=4\pi\sin\theta/\lambda$ ($\lambda$ is the wavelength of the X-ray radiation and $\theta$ is the scattering angle). The article of manufacture can be a film, a fiber, woven fibers, or nonwoven fiber web, absorbent articles (e.g., wipers, diapers, adult incontinence products, feminine pads), garments, protective fabrics and suits (e.g., surgical gowns or drapes, work overalls, dust or chemical protective outfits), wrapper or packaging materials or articles (e.g., diaper bag), face-masks, medical drapes, endotracheal tube, catheters, bladders or balloons, or any other item that may require a certain degree of flexibility or pliability and perceived and yet tougher. The article can be molded from or the film made from an extrusion of a thermoplastic composition described.

In another aspect, the present invention also pertains to a method of plasticizing a crystalline-phase-containing polymer, the method comprises: providing in a mixture a starting thermoplastic polymer with 14 to 25% crystallinity and a plasticizing agent having a xanthene-based molecular structure present in an amount of up to 5000-6000 ppm of total composition; mixing said thermoplastic polymer and said plasticizing agent in a molten or liquid state between a temperature range of 140-300°C; and dispersing uniformly said plasticizing agent throughout said molten mixture; and solidifying said molten mixture such that said xanthene-based molecular structure migrates into an amorphous phase; wherein the starting thermoplastic polymer is a polyalkylcarboxylic acid; and wherein the crystalline content of the polyalkylcarboxylic acid is computed from the crystallinity index:

$$X\% = \frac{I_c}{I_t} = \frac{\int i_c k^2 dk}{\int i_t k^2 dk} \quad (1)$$

where $I_c$ is the XRD intensity scattered from the crystalline portion of the material, $I_t$ is the total XRD intensity and k is the scattering vector with a magnitude $k=4\pi\sin\theta/\lambda$ ($\lambda$ is the wavelength of the X-ray radiation and $\theta$ is the scattering angle). When solidified the resulting solid exhibits a crystalline content that is at least 40 % less than the underlying or original thermoplastic polymer that was without the plasticizing agent. The method may further involve extruding or forming the molten mixture of the hot plasticized thermoplastic material into variety of solid forms or products when at about ambient room temperature.

Additional features and advantages of the xanthene containing semi-crystalline thermoplastic polymeric compositions will be described in the following detailed description. It is understood that the foregoing general description and the following details description and examples are merely representative of the invention, and are intended to provide an overview for understanding the invention as claimed.

BRIEF DESCRIPTION OF THE FIGURES

FIG. 1 shows a graph of the DSC curve of Control Sample (PLA control - solution cast); first heat.
FIG. 2 shows a graph of the DSC curve of Example #1 (PLA+5000 ppm DBF-solution cast); first heat.
FIG. 3 is a graph of the XRD curve of Control Sample; solution cast.
FIG. 4 is a graph of the XRD curve of Example #1; solution cast.
FIG. 5 is a graph of the DSC curve of Control Sample (PLA control); cooling and second heat.
FIG. 6 is a graph of the DSC curve of Example #1 (PLA+5000 ppm DBF); cooling and second heat.
FIG. 7 shows a graph of the XRD curve of Control Sample after 10°C/min cooling.
FIG. 8 shows a graph of the XRD curve of Example # after 10°C/min cooling.
FIG. 9 shows a graph of the XRD curve of Control Sample after slow (oven) cooling.
FIG. 10 shows a graph of the XRD curve of Example #1 after slow (oven) cooling.
FIG. 11 is a graph of the XRD experimental curve of Example #7, after cooling at 10°C/min (curve #1). Curves #2 and #3 are two amorphous halos and curves #4 and #5 are two crystalline peaks.
FIG. 12 shows the DMA changes of the storage modulus (E') of Control Sample (dashed curve) and Example #1 (solid curve) after cooling with 10°C/min.
FIG. 13 shows the stress-strain curves @ 40°C of Control Sample (PLA and 10°C/min cooling; solid circles) and Example #1 (PLA+5000ppm DBF and 10°C/min cooling; open circles).

DETAILED DESCRIPTION OF THE INVENTION

Section 1. - Definitions

In general, the present invention pertains to thermoplastic polymer compositions that are modified with a plasticizing compound containing a xanthene or xanthene-based molecular structure.

As used herein, the term "thermoplastic polymer" or "thermoplastic material" refers to an organic macromolecule composed of a large number of monomers, with molecular weight that may range from about 5,000 into the hundreds of thousands, which softens when exposed to heat and returns to its original condition when cooled to room temperature, such as polyalkylcarboxylic acids and more specifically polylactic acid.

As used herein, a "plasticizer," "plasticizing agent," or "plasticizing compound" is an organic compound that is added to a thermoplastic polymer which can both facilitate processing and increase the flexibility of the final product by modifying the molecular bonds of the polymer. Typically, the polymer molecule is held together by secondary valence bonds. The plasticizer replaces some of these bonds with plasticizer-to-polymer bonds, thus aiding movement of the polymer chain segments.

As used herein, a "xanthene" or "xanthene-based" molecule refers to an unmodified xanthene molecule or a derivative compound with a xanthene ring structure, as shown below. Xanthene ($CH_2(C_6H_4)_2O$) (dibenzopyran, tricyclic), a yellow organic heterocyclic compound, has the following chemical structure:

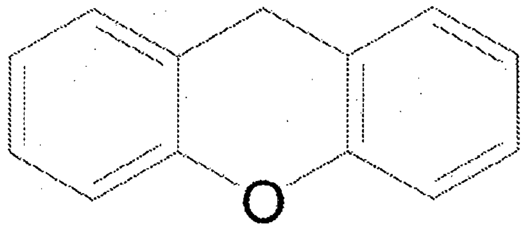

It is soluble in ether, and its melting point is 101-102 °C and its boiling point is 310-312°C. Xanthene is commonly used as a fungicide and is also a useful intermediate in organic synthesis. The xanthene molecule can be halogenated (F, Cl, Br, I). Halogenated xanthene structures may include, for example, mono-fluoro, di-fluoro, tri-fluoro, or tetra-fluoro-fluoresceins; mono-chloro, di-chloro, tri-chloro, tetra-chloro-fluorescein; mono-bromo, di-bromo, tri-bromo, or tetra-bromo-fluorosceins; or mono-iodo, di-iodo, tri-iodo, or tetra-iodo-fluoresceins, and mixtures thereof. Additionally, mixed halogenated xanthenes structures such as tetra-bromo-tetra-chloro-xanthene (e.g., Drug & Cosmetic (D&C) Red No.27), are also contemplated.

Section II. - Description

As the general public develops a wider social awareness of so-called "green" technologies and a desire to purchase products made from renewable materials, manufacturers are facing a challenge to try to respond to this consumer demand. Moreover, governmental requirements increasingly mandate the use of renewable or reusable materials in certain classes of disposable products has spurred a need to develop better and more innovative ways to deal with waste. In recent years manufacturers of plastic or thermoplastic products or materials have shown increasing interest in polylactic acid (PLA) polymers, which are an important, naturally occurring, renewable resource material that is inherently biodegradable. Since the material can be thermally processed into film, fibers, and molded parts, these polymers are a potential raw material replacement for polyolefins and other thermoplastic resins in consumer product and other applications. Manufacturers are seeking new ways to incorporate more recyclable or natural and biodegradable materials into otherwise conventional polymer-based products. Due to polylactic acid being both a sustainable and biodegradable or compostable polymer, interest grows in leveraging this "green" technology for a variety of uses, such as packaging, bottles, and disposable use articles.

The composting and biodegradation properties of semi-crystalline versus amorphous PLA materials differ. It is understood that the biodegradation kinetics of polylactic acid molecules are faster in the breaking down of amorphous regions and significantly slower in the breakdown more crystalline regions (Hideto Tsuji and Yoshito Ikada., J. Appl . Polym. Sci. 63: 855-863, 1997). Hence, it would be highly desirable to make essentially all of the polylactic acid be in an amorphous phase to increase the biodegradation kinetics of the polylactic acid waste. The composition according to the present invention describes such an essentially amorphous polylactic acid material.

In the past, the shortcoming associated with highly amorphous polymers is typically that the materials tended to be much weaker in strength/toughness compared to a more crystalline sample of the polymer. We have been able

to overcome this disadvantage. The polylactic acid of the present invention can be actually stronger or tougher than control samples of semi-crystalline PLA polymer. This feature is a unique and unexpected finding of this inventive composition. In other words, materials of the present composition are both highly amorphous and yet surprisingly tougher than their analogue semi-crystalline control materials. These properties provide an ideal polymer composition to meet the application needs such as describe above.

A.

In the past polylactic acid has been used as a specialty medical polymer at very high costs (> $10.00/g). Recent advances in polymer synthesis, and the use of renewable resources and agricultural waste products as feedstock's have made the production of this material at commodity prices possible. The literature on the polyester polymers derived from lactic acid can be somewhat confusing because there are several different methods for naming these polymers. Frequently, the abbreviation PLA refers not only to polymers derived from the "L" isomer of lactic acid (1) but also a mixture of the "L" isomer and the "D" isomer (2).

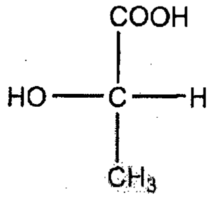

1

L-(+) lactic acid

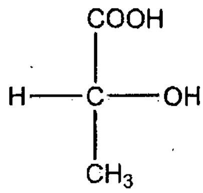

2

D-(-) lactic acid

In addition, some workers have named poly(lactic acid) polymers as polylactides because these polymers were prepared from "lactide" (3),the cyclic "dimer" of L-(+) lactic acid.

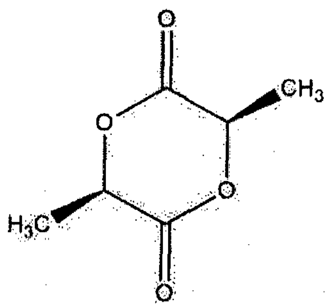

3

L,L-Lactide

Unless stated otherwise, PLA will refer generically to all polylactic acid polymers.

L-(+)-lactic acid is obtained by fermentation of inexpensive sources of glucose such as potato wastes, cheese whey permeate, or corn. The fermentation-derived L-lactic acid, however, is difficult to purify from all of the other carbohydrate by-products and bacterial cell breakdown products. The D-isomer of lactic acid is difficult to obtain, but researchers are exploring methods to genetically engineer bacteria to produce this isomer. The racemic mixture of lactic acid is synthesized from acetaldehyde. At this time, the racemic mixture is cheaper than the L-isomer which in turn is considerably cheaper than the D-isomer.

High molecular weight poly(L-lactic acid) is prepared by heating the lactide 3 in the presence of tin, lead, antimony or zinc catalysts, especially tetraphenyl tin[4] or stannous octoate (2-ethylhexanoate)[3a,b,d,5]. If the lactide is rigorously purified to remove water and trace of acids, high molecular weight poly(L-lactic acid) is obtained.

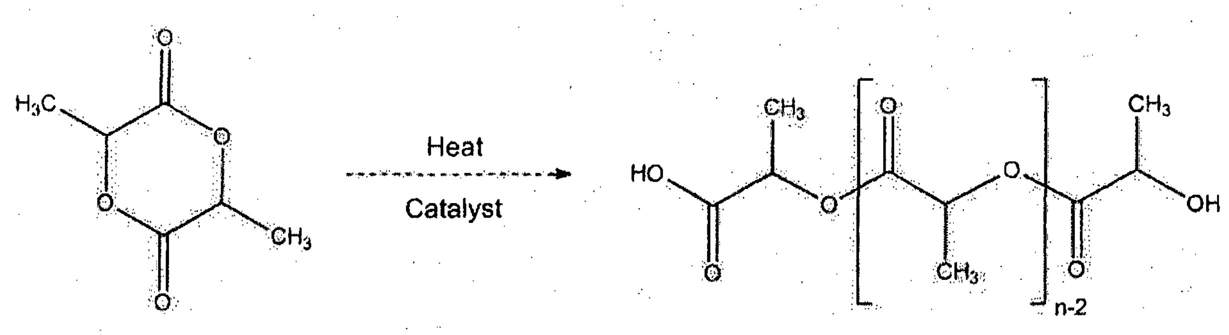


Lactide

High MW PLA

The resulting poly(L-lactic acid) [or poly(D-lactic acid) if D-lactic acid is used] is an isotactic, hydrophobic, brittle, tough polymer which becomes rubbery around 55-65°C (Tg). It is semi-crystalline (37%) with a broad range of melting points (Tm) from 173-215°C. Poly(D,L-lactic acid) is reported to be totally amorphous, atatic, inelastic, and glassy with a softening point of 53°C.

B.

Although some polymers may be completely amorphous, the morphology of most polymers is semi-crystalline. That is, they form a combination of crystalline and amorphous portions with the amorphous regions surrounding the crystalline areas. The mixtures of small crystals and amorphous material melt over a range of temperature instead of at a single melting point. The crystalline material tends to have highly ordered and regular structures formed by folding and stacking of the polymer chains. The amorphous structure, in contrast, shows no long range order, and have molecular chains are arranged randomly and in long chains which twist and curve around one-another, making large regions of highly structured morphology unlikely.

The highly ordered crystalline structure and amorphous morphology of certain polymer materials determine the differing behaviors of the polymer. An amorphous solid is formed when the chains have little orientation throughout the bulk polymer. The glass transition temperature (Tg) is the point at which the polymer hardens into an amorphous solid. The glass transition temperature of a polymer is an important factor in its physical properties and behavior for certain desired uses. As the temperature of a polymer drops below its Tg, the polymer behaves in an increasingly brittle manner; while, as the temperature rises above the Tg, the polymer becomes more viscous-like. In general, polymers with Tg values of well below room temperature (~20°C) define the domain of elastomers, and those with values above room temperature define rigid, structural polymers.

The Tg can influence the mechanical properties of the polymeric material; in particular, the response of the material to an application of a force, namely: *elastic* and *plastic behaviors.* Elastic materials will return to their original shape once the force is removed. Plastic materials will deform fluidly and not regain their shape. In plastic materials, flow is occurring, much like a highly viscous liquid. Most materials demonstrate a combination of elastic and plastic behavior, exhibiting plastic behavior after the elastic limit has been exceeded. For example, polyvinyl chloride (PVC) has a $T_g$ of 83°C, making it good, for example, for cold water pipes, but unsuitable for hot water. PVC also will always be a brittle solid at room temperature. Adding a small amount of plasticizer to PVC can lower the $T_g$ to about - 40°C. This addition renders the PVC a soft, flexible material at room temperature, ideal for applications such as garden hoses. A plasticized PVC hose can, however, become stiff and brittle in winter. In this case, as in any other, the relation of the $T_g$ to the ambient temperature is what determines the choice of a given material in a particular application.

In most polymers, the combination of crystalline and amorphous structures forms a material with advantageous properties of strength and stiffness. According to the present invention, while in furtherance of the work described in U.S. Patent Applications No. 11/974369, and No. 11/974393, we have discovered that xanthene or xanthene-based compounds can impart significant plasticizing properties to polyalkylcarboxylic acid materials with a crystalline level of 14% to 25% by weight of polymer. Examples of suitable xanthene-based compounds include xanthene dyes (e.g., xanthene base structure of fluorescein systems). Xanthene dyes are a class of dyes which includes fluoresceins, eosins, and rhodamines. They fall into three major categories: the fluorenes or amino xanthenes, the rhodols or aminohydroxyxanthenes, and the fluorones or hydroxy-xanthenes. Lillie, H. J. CONN's BIOLOGICAL STAINS, p.326 (Williams & Wilkins, 9th ed. 1977). Xanthene dyes tend to be fluorescent, yellow to pink to bluish red, brilliant dyes. The xanthene structure can have at least one functional R group, where R is hydrogen or halogen.

According to embodiments of the invention, xanthene and/or xanthene dyes can be incorporated into the thermoplastic polymer matrix by melt-mixing to enhance the physical plasticity of the resultant composition. Typically,

the molten mixture is heated to a temperature of between about 140°C and 280°C. This temperature can range from about 150°C or 180°C to about 230°C, 250°C or 265°C, depending on the melting temperatures of specific thermoplastic polymers.

Nonetheless, according to the present invention, not all xanthene-based structures function well as a plasticizer. We have found that xanthenes-based compounds with ketone or carboxylic acid analogues (e.g., xanthone and xanthene-carboxylic acid) do not work as well as others since they appear not to impart good plasticizing characteristics, but rather can make the polymer material very brittle, even worse than a control sample of the original thermoplastic polymer material.

According to the present invention, the starting thermoplastic polymer is a polyalkylcarboxylic acid with a crystalline content of 14% to 25% by weight of polymer. The plasticizing compound with a xanthene-based molecular structure can be in an amount from about 1000ppm to 6000ppm. Typically, the plasticizing compound with a xanthene-based molecular structure can be present in an amount from 3000ppm to 5000ppm, inclusive.

The starting thermoplastic polymer, according to the present invention, has a crystalline phase, and an amorphous phase in a ratio range of about 14-87:86-13 respectively (desirably, about 25:75 crystalline phase: amorphous phase). The crystalline phase of the composition comprising the starting polymer with the xanthene or xanthene-based structure is reduced by an amount of 40% up to 99% relative to the percentage of crystalline phase of an identical composition absent the plasticizing compound with xanthene-based molecular structure.

In the crystallization process, it has been observed that relatively short chains organize themselves into crystalline structures more readily than longer molecules. Therefore, the degree of polymerization (DP) is an important factor in determining the crystallinity index of a polymer. Polymers with a high DP have difficulty organizing into layers because they tend to become tangled. Low molecular weight polymers (short chains) are generally weaker in strength. Although they are crystalline, only weak Van der Waals forces hold the lattice together. This allows the crystalline layers to slip past one another causing a break in the material. High DP (amorphous) polymers, however, have greater strength because the molecules become tangled between layers. In the case of fibers, stretching to 3 or more times their original length when in a semi-crystalline state produces increased chain alignment, crystallinity and strength.

Also influencing the polymer morphology is the size and shape of the monomers' substituent groups. If the monomers are large and irregular, it is difficult for the polymer chains to arrange themselves in an ordered manner, resulting in a more amorphous solid. Likewise, smaller monomers, and monomers that have a very regular structure (e.g. rod-like) will form more crystalline polymers.

The cooling rate also influences the amount of crystallinity. Slow cooling provides time for greater amounts of crystallization to occur. As used herein, "slow cooling" or "oven cooling" refers to a process in which one heats up an oven, such as in the present situation a vacuum oven, to its maximum temperature (e.g., 300°C), place a sample in the oven, and turn off the heating element to allow the oven to gradually cool to ambient temperature. The oven cools down to room temperature over the course of several hours (e.g., 4-7 hrs.). To illustrate, for example, the maximum temperature is 225°C (above the melting of PLA) and the cooling time is 4-5 hours. This cooling time is compared to the cooling with a rate of 10 C/min, which informs that the cooling time is about 22.5 minutes.

Fast cooling rates, on the other hand, such as rapid quenches, yield highly amorphous materials. Subsequent annealing (heating and holding at an appropriate temperature below the crystalline melting point, followed by slow cooling) will produce a significant increase in crystallinity in most polymers, as well as relieving stresses.

C.

To reiterate, the present invention, in part, relates to a thermoplastic polymer composition. The composition includes: a starting base semi-crystalline polymer with a minimal crystalline content of 14% to 25% by weight of the polymer, and a plasticizing compound with a xanthene-based molecular structure in an amount up to 6000 ppm dispersed therein, and said blended composition mixture having a crystalline phase, and an amorphous phase in a ratio range of 0-15:85-100, respectively when solid at ambient room temperature. The plasticizing compound is dispersed within the amorphous phase and the semi-crystalline polymer is a polyalkylcarboxylic acid. The blended composition may have a level of crystallinity of at least 40-99% less than a starting control thermoplastic polymer not containing the plasticizing compound.

The composition has a ratio of 0-15:85-100, respectively of the crystalline phase to amorphous phase when solid. The semi-crystalline polymer contains a crystalline content of 0% to 15% crystalline phase, 100% to 85% amorphous state. The semi-crystalline polymer is selected from a group consisting: polyalkylcarboxylic acids, in particular polylactic acid.

The plasticizing compound may have at least one R group, where R is hydrogen or a halogen. In other words, the xanthene-based molecular structure can be halogenated or mixed-halogenated. The plasticizing compound may be present at 3000 ppm to 5000 ppm. The crystalline phase is reduced by an amount of 40% to 100% or up to 300-400-500%, relative to the percentage of crystalline phase of an identical composition absent the plasticizing compound.

The composition can increase the toughness relative to an underlying thermoplastic polymer not having the

plasticizing compound by at least 40% to 60%. The composition increases in the stretch elongation tolerance relative to an underlying thermoplastic polymer not having plasticizing compound by at least 40% to 50%. The composition exhibits an increase in the strain at break said polymer relative to an underlying thermoplastic polymer not having plasticizing compound by at least 40%. The composition exhibits an increase in the stress at break said polymer relative to an underlying thermoplastic polymer not having plasticizing compound by at least 40%.

The composition exhibits faster biodegradation and composting kinetics compared to a control sample of the same polymer without the xanthene or xanthenes-based additive and yet is tougher than the control polymer without the xanthenes or xanthene-based additive. The composition is essentially amorphous and tougher than the control polymer without the xanthenes or xanthene-based additive.

According to another aspect, the invention discloses a method of plasticizing a crystalline-phase-containing polymer. The method involves: providing a starting thermoplastic polymer with 14 to 25% crystallinity index phase. The method comprises: providing in a mixture a starting polymer with 14% to 25% crystallinity and a plasticizing agent having a xanthene-based molecular structure present in an amount of up to 5000-6000 ppm, of total composition; mixing said thermoplastic polymer and said plasticizing agent in a molten or liquid state between a temperature range of 140°C to 300°C - typically, 140°C or 150 °C up to about 285°C or 290°C; and dispersing uniformly said plasticizing agent throughout said molten mixture; and solidifying said molten mixture such that said xanthene-based molecular structure migrates into an amorphous phase. One may form an article from the molten mixture. The method may further include extruding or forming said molten mixtures into a solid when at about ambient room temperature. When solidified, the resulting solid exhibits a level of crystallinity that is at least 40% to about 100% or 500% less than original thermoplastic polymer without said plasticizing agent. The mixture is heated to a temperature of between 170°C and 280°C.

Section III. - Practical Applications

In another aspect, the invention describes an article of manufacture that is fabricated with least in part with a thermoplastic polymer. The thermoplastic polymer has a semi-crystalline polymer matrix incorporating a plasticizer comprising a xanthene molecule or a compound with a xanthene-based molecular structure. The xanthene molecule or compound with a xanthene-based molecular structure is present in the polymer matrix in an amount of 3000 ppm up to 5000 ppm. The xanthenes molecule or compound with a xanthene-based molecular structure may be selected from an unmodified, halogenated, or mixed-halogenated xanthenes-based compound. The underlying original thermoplastic polymer is selected from a group consisting: polyalkylcarboxylic acids. The article can be a fiber or filament, or fiber-web made from an extrusion of said thermoplastic composition comprising a starting semi-crystalline polymer with a minimal crystalline content of 14% by weight of the polymer, and a compound with a xanthene-based molecular structure in an amount up to 5000-6000 ppm dispersed therein, and said blended final composition having a crystalline phase, and an amorphous phase in a ratio range of 0-15:85-100, respectively when solid at ambient room temperature. The article may the form of a film, fiber, fiber web, absorbent pad, diaper, adult incontinence or feminine hygiene product, protective fabric, face-mask, medical drape, wiper, garment, and packaging article. In such an embodiment, the fiber can be part of a woven fabric. The fiber-web can form part of a nonwoven fabric. The article can be a laminate structure with a film layer made from an extrusion of the thermoplastic composition.

In general, according to the present invention, thermoplastic polymers compositions that have xanthene-based compounds incorporated tend to be tougher at a temperature range of about 30°C to about 50°C, which appears to be an effect of lowering the crystalline content of the modified or plasticized polymer. Incorporation of the plasticizer compound into the thermoplastic polymeric composition can increase the relative toughness of the base or underlying thermoplastic polymer by about at least 20%. In certain embodiments, the toughness can be enhanced by as much as 20% up to about 50%. Strain at break of a xanthene coantining polymer sample is increase compared to the control by at least 20% to 40%. The relative tolerance for elongation is improved by about at least 20%. This parameter can also be increased by about 20%, up to about 50%. The xanthenes containing thermoplastic material exhibits an increase in stress to break applied to pull apart the polymer by at least 20%, and up to about 50%.

With such properties, films, fibers, and fibers-webs formed from the present compositions tend to be more drapable and ductile. This chracteristic would allow manufacturers to provide garments, covers, wrapping materials, or packages made with woven or nonwoven fabric materials made with such polymers to be more conformable and convey a softer texture to the touch. Garments may include, for example, overalls, gowns, drapes, footwear, gloves, or headwear. Also a more pliable quality of the polymer can result in a quieter film and less "crinkle in noise" when crumbled or crushed. Fibers and fabrics made from those fibers containing the xanthenes-based compounds would be softer and also more drapable. Thus for example, polypropylene nonwoven would be converted into a more polyethylene-like softer fabric (less harsh in feel). This property would be ideal for manufactured articles that incorporate nonwoven layers, especially for those that contact a consumer or user's skin, such as absorbent pads, feminine hygiene pads, diapers, or wash cloths and wipers. In particular, manufacturers can modify existing nonwoven technology and materials, for instance, co-formed fibers or hydroknit fibers, combined films, fibers and webs for laminate structures, such as sponbond, sponbond-meltblown-spunbond, sponbond-film-sponbond. A film can allow for micro-porous pore dimensions of about 10-50-100 microns. Other potential products may include injection or extrusion molded articles, for example, bladders or balloons, catheter tubing, or endotracheal tubes. Conventionally, such tubes and conduits have been made from rather rigid thermoplastic polymers, which may cause pain and tissue damage, if not inserted smoothly along the passageways in a patient's body. A more pliable material that is able to flex when contacting a bend, for instance, in the trachea or esophagus would avoid such injuries. Therefore the uniqueness of this invention is the ability to convert "harder polymers" into softer and more pliable films, fibers, webs made from the fibers, and/or laminate structures.

Section IV. - Empirical

**1. Materials**

All chemicals and solvents were obtained from Sigma-Aldrich Chemical Company (Milwaukee WI) and used without further purification unless specified in the following section.

**2. Analytical Methods**

DSC

The samples were analyzed on a TA Instruments DSC Q 2000 (T-zero Cell) using the following experimental procedure:

Approximately 5 mg cut from the specimens were encapsulated in the DSC pans. The specimens were run in the temperature interval 20°C to 200°C with a heating/cooling rate of 10°C/min. The as-received solution cast materials and the materials melted and slowly cooled (cooling time 4-5 hours) in a vacuum oven were tested. All measurements were executed in an inert gas (He) atmosphere.

WAXS

The materials were analyzed on an X-ray diffractometer D-max Rapid from Rigaku Corp. equipped with a two dimensional (2-D) position sensitive detector. The measurements were executed in transmission geometry and Cu Kα radiation (λ = 1.5405 Angstrom). The as-received solution cast materials, the materials obtained after melting and re-crystallization in the DSC pans (cooling rate 10°C/min) and the materials melted and slowly cooled (cooling time 4-5 hours) in a vacuum oven were tested.

DMA

Films with thickness ~0.25mm were compression molded at 225°C and cooled to room temperature with a rate of 10°C/min. Strips with lengths 10 mm and widths 3 mm were studied using Q800 instrument from TA Instruments. The samples were tested in tension/tension deformation mode. The experimental runs were executed in a temperature sweep mode in the range from -30°C to +50°C with a heating rate of 3°C/min, at constant frequency (2 Hz) and constant static force of 2.5N. The stress amplitude was ±10% of the static force.

Crystallinity Index

The crystalline content of any semicrystalline material can be computed from the crystallinity index)[29]

$$X\% = \frac{I_c}{I_t} = \frac{\int i_c k^2 dk}{\int i_t k^2 dk} \quad (1)$$

Where $I_c$ is the XRD intensity scattered from the crystalline portion of the material, $I_t$ is the total XRD intensity and k is the scattering vector with a magnitude $k=4\pi\sin\theta/\lambda$ (λ is the wavelength of the X-ray radiation and θ is the scattering angle).

### 3. Sample Preparation

Example 1. 4, 5-Dibromofluorescein (DBF, 5000ppm) in polylactic acid

An amount of 1.0 gram polylactic acid (molecular weight Mn -30,000; Mw -55,000) was dissolved in 20ml of chloroform at ambient temperature with stirring. The clear solution was separated into two equal 10ml aliquots and to one of which was added 100mg of dibromofluorescein and the mixture stirred to dissolve the dye. Both solutions were solvent cast by pouring into respective aluminum weighing pans (6cm diameter x 1cm depth) and the solvent evaporated overnight (8-12 hrs.) in a fume hood. The films were then removed and analyzed.

Example 2. 4, 5-dichlorofluorescein (DCF, 5000ppm) in polylactic acid

The procedure described in example 1 was repeated except that dichlorofuorescein was used.

Example 3. 4, 5-diiodofluorescein (DIF, 5000ppm) in polylactic acid

The procedure described in example 1 was repeated except that diiodofluorescein was used.

Example 4. Xanthene (X, 5000ppm) in polylactic acid

The procedure described in example 1 was repeated except that xanthene was used.

Example 5. Tributyl-o-acetylcitrate (TBAC, 5000ppm) in polylactic acid

The procedure used in example 1 was repeated except that TBAC was used.

Example 6. 4,5-Dibromofluoroscein (BDF, 2000ppm) in polylactic acid

The procedure used in example 1 was repeated except that 40mg of dibromofluoroscein was used.

Example 7. 4,5-Dibromofluorescein (DBF, 7000ppm) in polylactic acid

The procedure used in example 1 was repeated except that 350mg of dibromofluoroscein was used.

### 4. Results

In Figures 1 and 2, the DSC heating curves - I$^{st}$ Heat of the two materials are drawn. Inspection of Figure 1, shows that the glass transition process is clearly detectable for Sample#1, while it is quite smeared or indiscernible for Sample#2 in Figure 2. Further, Sample#1 is characterized with a significant cold crystallization process, while Sample#2 is not. These results are indicative of relatively low crystallinity (as measure by its crystallinity index) content in Sample#1 (the solution cast control sample) and a much higher level of crystallinity (crystallinity index) in Sample#2 (the solution cast PLA+5000ppm DBF). XRD results shown in Figures 3 and 4, confirm the DSC results. After fitting the experimental X-ray curves into crystalline and amorphous components it was found that the crystalline fraction is ~14% in Sample#1 vs. 52% in Sample#2, as summarized in Table 1.

TABLE 1

| Sample ID | X% after Solution Casting | X% after Cool 10°C/min | X% after Slow Cool |
|---|---|---|---|
| Control Sample PLA control | 14 | 25 | 87 |
| Example #1 PLA+5000ppm DBF | 52 | 0 | 85 |

These results would tend to suggest that the DBF acts as a nucleating agent in the PLA matrix. The cooling DSC curves and the II$^{nd}$ Heat curves (Figures 5 & 6), however, indicate exactly the opposite. The molten Sample#1 crystallizes partially after cooling with a rate of 10°C/min, while Sample#2 appears to lack any crystallinity under the same conditions. The XRD curves (Figures 7 & 8) confirm the DSC results and the fitting gives ~25% crystallinity index vs. ~0% in Table 1. After melting and slow cooling both materials are characterized with a very high crystalline index content as illustrated by Figures 9 and 10, and Table 1.

**Other xanthene compounds**

Other xanthenes compounds were tested in the PLA. Table 2, below, reports that all the xanthenes compounds have the similar unique effect on PLA as the DBF, however DBF appears to have the largest effect. The TBAC sample was made to compare the xanthenes to the traditional plasticizer. The results show that TBAC reduces the crystallinity (as measured by its crystallinity index) after the cool cycle, but not as great as the xanthenes.

TABLE 2 - Percent Crystalline Index

| Sample | X% after solution casting | X% after cool 10C/min | X% after Slow cool |
|---|---|---|---|
| Control Sample PLA control | 14 | 25 | 87 |
| Example #1 PLA +5000ppm DBF | 52 | 0 | 87 |
| Example #2 PLA + 5000ppm X | 38 | 14 | 85 |
| Example #3 PLA + 5000ppm DCF | 43 | 11 | 86 |
| Example #4 PLA + 5000ppm DIF | 41 | 4 | 83 |
| Example #5 PLA + 5000ppm TBAC | 14 | 15 | N/A |
| Example #6 PLA + 2000ppm DBF | 6 | 8 | N/A |
| Example #7 PLA + 7000ppm DBF | 5 | 4 | N/A |

**Explanation of the Analysis of the XRD Curve**

By way of illustration, the XRD experimental curve of Example #7, PLA containing 7000 ppm DBF, after slow cooling at 10°C/min (curve #1) is shown in Figure 11. The curve has been analyzed to show the composition of both crystalline and amorphous components of the PLA polymer. Curve # in Figure 11 represents the composite curve of the other curves #2-5. Curves #2 and #3 are two amorphous halos, and curves #4 and #5 are two crystalline peaks. The crystalline index of the resulting material from Example #7 is reduced by a factor of 4-5 when compared to the crystallinity of its starting polymer material. It is believed that this results from the incorporation of DBF to generate an increased amorphous state content in the material. More thorough blending of the xanthenes-like molecules throughout the starting polymer materials may generate even better results.

**The DMA Study of the Storage Modulus of PLA control versus PLA with 5wt % DBF with Temperature**

Films with thickness ~0.25mm were compression molded at 225°C and cooled to room temperature with a rate of 10°C/min. Strips with lengths 10 mm and widths 3 mm were studied using Q800 instrument from TA Instruments. The samples were tested in tension/tension deformation mode. The experimental runs were executed in a temperature sweep mode in the range from -30°C to +50°C with a heating rate of 3°C/min, at constant frequency (2 Hz) and constant static force of 2.5N. The stress amplitude was ±10% of the static force.

The DMA analysis of the storage modulus of PLA control film versus PLA containing 5000 ppm DBF over temperature shows a most unusual and unique property. Figure 12 shows the remarkable curves. It would appear that the DBF sample maintains good storage modulus across the temperature range (-30°C to 50°C) without softening. This is surprising considering it is essentially amorphous and should soften with increasing temperature. In contrast, the control PLA softens dramatically at around 10°C with a large decrease in storage modulus from about 30°C and higher. This is surprising as the sample that has some crystallinity (approximately 25% based on its crystallinity index). Clearly, the results are the opposite of what one skilled in the art would expect and predict. While the mechanism of the action of DBF is not fully understood, it is thought that the DBF is acting, in some manner, as a mild chain linking agent of the amorphous polymer chains resulting in not allowing the chains to move about with the same freedom of motion that the control amorphous chains express.

TABLE 3 - Storage Modulus at Different Temperatures

| Temperature Sample ID | -25°C | -10°C | 0°C | 5°C | 10°C | 15°C | 20°C | 25°C | 30°C | 35°C | 40°C | 45°C |
|---|---|---|---|---|---|---|---|---|---|---|---|---|
| E' - PLA Control | 2.2 | 2.1 | 2.03 | 1.99 | 1.95 | 1.91 | 1.87 | 1.82 | 1.73 | 1.61 | 1.3 | 1.09 |

(continued)

| | | | | | | | | | | | | |
|---|---|---|---|---|---|---|---|---|---|---|---|---|
| E' - PLA + 5000ppm DBF | 2.1 | 2.05 | 2.02 | 2 | 1.99 | 1.97 | 1.96 | 1.945 | 1.93 | 1.93 | 1.92 | 1.89 |
| Note: The values of the storage modulus in Table II are in MPaX$10^{-3}$. | | | | | | | | | | | | |

**Analysis of the stress-strain (toughness) of the films samples**

Sample ID

Control Sample - PLA control 1 (solution cast)

Example #1 - PLA + 5000ppm Di-Bromo Fluorescein (DBF - solution cast)

Preparation of the film samples for DMA analysis

Films with a thickness of ~0.25 mm were compression molded at 225°C and cooled to room temperature with a rate of 10°C/min. (The oven of the DMA instrument was utilized for the controlled cooling.) Strips with lengths 20 mm and widths 3 mm were studied using Q800 instrument from TA Instruments. The samples were tested in Stress-Strain deformation mode at temperatures 25°C and 40°C. The applied force was increase with a rate of 2 N/min until the break of the tested specimens.

In Figure 13 are plotted the stress-strain curves of the two materials at temperature 40°C. The results indicate that the modified material is characterized with higher elongation and stress at break. The relevant parameters are summarized in Table 4.

TABLE 4 - Deformation @ 40°C

| Sample ID | $\varepsilon_b$[%] | $\sigma_b$[MPa] | W[MPa] |
|---|---|---|---|
| PLA | 0.73 | 2 | 1.1 |
| PLA+ 500ppm DBF | 1.13 | 4 | 3.3 |
| Note: In Table 4 $\varepsilon_b$ is the strain at break; $\sigma_b$ is the stress at break and W is the toughness (the area under the respective curves). | | | |

Overall, the results from the XRD and DSC studies indicate that the addition of a relatively small amount (e.g., about 5000 ppm) of DBF can affect significantly the crystallization behavior of PLA. The preparation of the materials by solution casting (similar in many respects to slow cooling) suggests that the DBF acts as a nucleating agent and increases significantly the crystallinity content (as measure by its crystallinity index, X%). However after melting and cooling with 10°C/min the DBF completely suppresses the crystal formation. Slow (oven) cooling is not affected by the presence of DBF. The other xanthenes also show this unique effect on PLA, but are not as functionally effective as the DBF system.

A surprising observation was made when the films were analyzed by DMA. The DBF-PLA films, while essentially amorphous, had essentially no change in their storage modulus on heating from -10°C to 30°C. Whereas the control PLA films, with approximately 25% crystallinity index, lost their storage modulus with increasing temperature with a steep slope after 20°C and higher. This is truly the opposite that anyone skilled in the art would have predicted based on the literature of semi-crystalline versus amorphous polymers.

After DMA analysis the samples were placed in chloroform at ambient temperature to see if they would dissolve in the solvent. If the DBF had cross-linked the polymer chains it would have made the films less likely to dissolve in the solvent. Both film samples were found to dissolve completely in the solvent showing that the polymer chains were not cross-linked. This makes the action of the DBF quite unique in its ability to suppress crystallization but make the essentially amorphous PLA film tougher, which is a surprising or unexpected, and unique finding.

While the mechanism is not fully understood, it is believed that the DBF is acting as a mild chain linking agent between the amorphous polymer chains restricting their motion as compared to the control amorphous polymer chains. Although some literature suggest that the addition of certain known cross-linking agents can strengthen polylactic acid, with the higher the concentration of crosslinker added the stronger the material, but xanthenes are not known to be cross-linkers. Xanthene or xanthene-like molecules do not have any suitable chemical sites that could participate in chemical crosslinking. With such unique properties (essentially amorphous and strong) the polymer composition of the present invention potentially may leads to its use in various applications. For instance, to fabricate articles that exhibit or are desired to have higher biodegradation/composting rates due to the essentially amorphous nature of the composition. Alternatively, it may be used for essentially amorphous polymer articles and yet tougher than the control or base

polymers in the temperature range from 30 to 50°C. The article of manufacture can be a film, a fiber, woven fibers, or nonwoven fiber web, absorbent articles (e.g., wipers, diapers, adult incontinence products, feminine pads), garments, protective fabrics and suits (e.g., surgical gowns or drapes, work overalls, dust or chemical protective outfits), wrapper or packaging materials or articles (e.g., diaper bag), face-masks, medical drapes, endotracheal tube, catheters, bladders or balloons, or any other item that may require a certain degree of flexibility or pliability and yet tougher.

## Claims

1. A thermoplastic polymer composition comprising: a starting base semi-crystalline polymer with a minimal crystalline content of 14% to 25% by weight of the polymer, and a plasticizing compound with a xanthene-based molecular structure in an amount up to 6000 ppm, preferably at 3000 ppm to 5000 ppm, dispersed therein, and said blended composition mixture having a crystalline phase, and an amorphous phase in a ratio range of 0-15:85-100, respectively when solid at ambient room temperature, wherein said plasticizing compound is dispersed within said amorphous phase; wherein said semi-crystalline polymer is a polyalkylcarboxylic acid; and wherein the crystalline content of the polyalkylcarboxylic acid is computed from the crystallinity index:

$$X\% = \frac{I_c}{I_t} = \frac{\int i_c\, k^2 dk}{\int i_t k^2 dk} \quad (1)$$

where $I_c$ is the XRD intensity scattered from the crystalline portion of the material, It is the total XRD intensity and k is the scattering vector with a magnitude k=4πsinθ/λ (λ is the wavelength of the X-ray radiation and 6 is the scattering angle).

2. The thermoplastic polymer composition according to claim 1, wherein said blended composition yields or exhibits a level of crystallinity of at least 40-99% less than an initial control thermoplastic polymer without said plasticizing compound with a xanthene-based molecular structure.

3. The thermoplastic polymer composition according to claim 1, wherein said plasticizing compound has at least one R group, where R is hydrogen or a halogen.

4. The thermoplastic composition according to claim 1, wherein said xanthene-based molecular structure is halogenated or mixed-halogenated.

5. The thermoplastic polymer composition according to claim 1, wherein said crystalline phase is reduced by an amount of 40% to 500% relative to the percentage of crystalline phase of an identical composition absent said plasticizing compound with xanthene-based molecular structure; or wherein said composition can increase the toughness relative to an underlying thermoplastic polymer not having said plasticizing compound by at least 40% to 60%; or
wherein said composition increases in the stretch elongation tolerance relative to an underlying thermoplastic polymer not having plasticizing compound by at least 40% to 50%; or wherein said composition exhibits an increase in the strain at break said polymer relative to an underlying thermoplastic polymer not having plasticizing compound by at least 40%; or
wherein said composition exhibits faster biodegradation and composting kinetics compared to the control polymer without the xanthene or xanthenes-based additive and yet is tougher than the control polymer without the xanthenes or xanthene-based additive; and wherein said composition is essentially amorphous and tougher than the control polymer without the xanthenes or xanthene-based additive;
wherein the toughness, stretch elongation and strain at break are measured on a film with a thickness of 0.25 mm, length of 20 mm and width of 3 mm, said film being compression moulded at 225°C and cooled to room temperature with a rate of 10°C/min, and tested at 40°C with the applied force increased at a rate of 2 N/min until break.

6. An article of manufacture comprising a thermoplastic polymer, the thermoplastic polymer having a semi-crystalline polymer matrix incorporating a plasticizer comprising a xanthene molecule or a compound with a xanthene-based molecular structure, said semi-crystalline polymer contains a crystalline content of 0% to 15% crystalline phase, 100% to 85% amorphous state, said xanthene molecule or compound with a xanthene-based molecular structure is present in said polymer matrix in an amount of 3000 ppm up to 5000 ppm; wherein said semi-crystalline polymer is a polyalkylcarboxylic acid; and wherein the crystalline content of the polyalkylcarboxylic acid is computed from

the crystallinity index:

$$X\% = \frac{I_c}{I_t} = \frac{\int i_c k^2 dk}{\int i_t k^2 dk} \quad (1)$$

where $I_c$ is the XRD intensity scattered from the crystalline portion of the material, It is the total XRD intensity and k is the scattering vector with a magnitude k=4πsinθ/λ (λ is the wavelength of the X-ray radiation and 6 is the scattering angle).

**7.** The article according to claim 6, wherein said xanthenes molecule or compound with a xanthene-based molecular structure is selected from an unmodified, halogenated, or mixed-halogenated xanthenes-based compound

**8.** The thermoplastic polymer composition according to claim 1 or the article according to claim 6, wherein said semi-crystalline polymer is polylactic acid.

**9.** The article according to claim 6, wherein said article is a fiber or filament, or fiber-web, preferably wherein said fiber is part of a woven fabric or said fiber-web forms a nonwoven fabric, made from an extrusion of said thermoplastic composition comprising a starting semi-crystalline polymer with a minimal crystalline content of 14% by weight of the polymer, and a compound with a xanthene-based molecular structure in an amount up to 5000-6000 ppm dispersed therein, and said blended final composition having a crystalline phase, and an amorphous phase in a ratio range of 0-15:85-100, respectively when solid at ambient room temperature.

**10.** The article according to claim 6, wherein said article is a laminate structure comprising a film layer made from an extrusion of said thermoplastic composition.

**11.** The article according to claim 6, wherein the article is: a) a film, fiber, fiber web, absorbent pad, diaper, adult incontinence or feminine hygiene product, protective fabric, face-mask, medical drape, wiper, garment, and packaging article; or b) an endotracheal tube, catheter tubing, a bladder or balloon.

**12.** A method of plasticizing a crystalline-phase-containing polymer, the method comprises: providing in a mixture a starting thermoplastic polymer with 14 to 25% crystallinity and a plasticizing agent having a xanthene-based molecular structure present in an amount of up to 5000-6000 ppm of total composition; mixing said thermoplastic polymer and said plasticizing agent in a molten or liquid state between a temperature range of 140-300°C; and dispersing uniformly said plasticizing agent throughout said molten mixture; and solidifying said molten mixture such that said xanthene-based molecular structure migrates into an amorphous phase; wherein the starting thermoplastic polymer is a polyalkylcarboxylic acid; and wherein the crystalline content of the 3polyalkylcarboxylic acid is computed from the crystallinity index:

$$X\% = \frac{I_c}{I_t} = \frac{\int i_c k^2 dk}{\int i_t k^2 dk} \quad (1)$$

where $I_c$ is the XRD intensity scattered from the crystalline portion of the material, It is the total XRD intensity and k is the scattering vector with a magnitude k=4πsinθ/λ (λ is the wavelength of the X-ray radiation and θ is the scattering angle).

**13.** The method according to claim 12, wherein further comprises extruding or forming said molten mixtures into a solid when at about ambient room temperature.

**14.** The method according to claim 12, wherein when solidified the resulting solid exhibits a level of crystallinity that is at least 40% up to 500 % less than original thermoplastic polymer without said plasticizing agent.

**15.** The method according to claim 12, wherein said mixture is heated to a temperature of between 170°C and 280°C.

## Patentansprüche

1. Eine thermoplastische Polymerzusammensetzung aufweisend: ein teilkristallines Ausgangsbasispolymer mit einem minimalen Kristallinitätsanteil von 14 bis 25 Gewichtsprozent des Polymers und eine darin dispergierte Weichmachverbindung mit einer Xanthen-basierten Molekularstruktur in einer Menge von bis zu 6000 ppm, bevorzugt von 3000 ppm bis 5000 ppm, und die besagte gemischte Zusammensetzungsmischung hat eine kristalline Phase beziehungsweise eine amorphe Phase in einem Verhältnisbereich von 0 - 15 : 85 - 100, wenn sie bei Umgebungsraumtemperatur fest ist, wobei die besagte Weichmachverbindung innerhalb der besagten amorphen Phase dispergiert ist, wobei das besagte teilkristalline Polymer eine Polyalkylcarbonsäure ist und wobei der Kristallinitätsanteil der Polyalkylcarbonsäure aus dem Kristallinitätsindex berechnet wird:

$$X\% = \frac{I_c}{I_t} = \frac{\int i_c k^2 dk}{\int i_t k^2 dk} \quad (1)$$

wobei $I_c$ die XRD-Intensität gestreut von dem kristallinen Teil des Materials ist, $I_t$ die gesamte XRD-Intensität ist und k der Streuvektor mit einer Magnitude $k=4\pi\sin\theta/\lambda$ ist ($\lambda$ ist die Wellenlänge der Röntgenstrahlung und 6 ist der Streuwinkel).

2. Die thermoplastische Polymerzusammensetzung gemäß Anspruch 1, wobei die besagte gemischte Zusammensetzung einen Kristal-linitätsgrad von zumindest 40 - 99 % weniger als ein thermoplastisches Anfangskontrollpolymer ohne die besagte Weichmachverbindung mit einer Xanthen-basierten Molekularstruktur hervorbringt oder aufweist.

3. Die thermoplastische Polymerzusammensetzung gemäß Anspruch 1, wobei die besagte Weichmachverbindung zumindest eine R-Gruppe hat, wobei R Wasserstoff oder ein Halogen ist.

4. Die thermoplastische Polymerzusammensetzung gemäß Anspruch 1, wobei die besagte Xanthen-basierte Molekularstruktur halogeniert oder gemischt halogeniert ist.

5. Die thermoplastische Polymerzusammensetzung gemäß Anspruch 1, wobei die besagte kristalline Phase um einen Betrag von 40 % bis 500 % relativ zu dem Prozentsatz von einer kristallinen Phase einer identischen Zusammensetzung ohne die besagte Weichmachverbindung mit Xanthen-basierter Molekularstruktur reduziert ist oder wobei die besagte Zusammensetzung die Zähigkeit relativ zu einem zugrundeliegenden thermoplastischen Polymer, welches die besagte Weichmachverbindung nicht hat, um zumindest 40 % bis 60 % erhöhen kann; oder
wobei die besagte Zusammensetzung sich in der Stretchdehnungstoleranz relativ zu einem zugrundeliegenden thermoplastischen Polymer, welches die Weichmachverbindung nicht hat, um zumindest 40 % bis 50 % erhöht oder wobei die besagte Zusammensetzung relativ zu einem zugrundeliegenden thermoplastischen Polymer, welches die Weichmachverbindung nicht hat, eine Erhöhung in der Bruchdehnung des besagten Polymers um zumindest 40 % aufweist; oder
wobei die besagte Zusammensetzung einen schnelleren biologischen Abbau und Kompostierkinetik verglichen mit dem Kontrollpolymer ohne das Xanthen oder Xanthene-basiertes Additiv aufweist und sogar zäher als das Kontrollpolymer ohne die Xanthene oder Xanthen-basiertes Additiv ist; und
wobei die besagte Zusammensetzung im Wesentlichen amorph und zäher als das Kontrollpolymer ohne die Xanthene oder Xanthen-basiertes Additiv ist;
wobei die Zähigkeit, Stretchdehnung und Bruchdehnung bei einem Film mit einer Dicke von 0,25 mm, Länge von 20 mm und Breite von 3 mm gemessen werden, wobei der besagte Film bei 225 °C formgepresst und mit einer Geschwindigkeit von 10 °C/Minute auf Raumtemperatur abgekühlt wird und bei 40 °C getestet wird, wobei die aufgewandte Kraft mit einer Geschwindigkeit von 2 N/Minute bis zum Bruch erhöht wird.

6. Ein Herstellungsgegenstand aufweisend ein thermoplastisches Polymer, das thermoplastische Polymer hat eine teilkristalline Polymermatrix umfassend einen Weichmacher aufweisend ein Xanthen-Molekül oder eine Verbindung mit einer Xanthen-basierten Molekularstruktur, das besagte teilkristalline Polymer beinhaltet einen Kristallinitätsanteil von 0 % bis 15 % kristalline Phase, 100 % bis 85 % amorpher Zustand, das besagte Xanthen-Molekühl oder Verbindung mit einer Xanthen-basierten Molekularstruktur ist in der besagten Polymermatrix in einer Menge von 3000 ppm bis zu 5000 ppm vorhanden, wobei das besagte teilkristalline Polymer eine Polyalkylcarbonsäure ist und wobei der Kristallinitätsanteil der Polyalkylcarbonsäure aus dem Kristallinitätsindex berechnet wird:

$$X\% = \frac{I_c}{I_t} = \frac{\int i_c\, k^2 dk}{\int i_t k^2 dk} \quad (1)$$

wobei $I_c$ die XRD-Intensität gestreut von dem kristallinen Teil des Materials ist, $I_t$ die gesamte XRD-Intensität ist und k der Streuvektor mit einer Magnitude k=4πsinθ/λ ist (A ist die Wellenlänge der Röntgenstrahlung und 6 ist der Streuwinkel).

**7.** Der Gegenstand gemäß Anspruch 6, wobei das besagte Xanthene-Moleküh1 oder Verbindung mit einer Xanthen-basierten Molekularstruktur ausgewählt wird aus einer unmodifizierten, halogenierten oder gemischt halogenierten Xanthene-basierten Verbindung.

**8.** Die thermoplastische Polymerzusammensetzung gemäß Anspruch 1 oder der Gegenstand gemäß Anspruch 6, wobei das besagte teilkristalline Polymer Polymilchsäure ist.

**9.** Der Gegenstand gemäß Anspruch 6, wobei der besagte Gegenstand eine Faser oder Filament oder Fasergewebe ist, bevorzugt wobei die besagte Faser Teil von einem Stoff ist oder das besagte Fasergewebe ein Faservlies bildet, hergestellt aus einer Extrusion der besagten thermoplastischen Zusammensetzung aufweisend ein teilkristallines Ausgangspolymer mit einem minimalen Kristallinitätsanteil von 14 Gewichtsprozent des Polymers und eine darin dispergierte Verbindung mit einer Xanthen-basierten Molekularstruktur in einer Menge von bis zu 5000 - 6000 ppm, und die besagte gemischte finale Zusammensetzung hat eine kristalline Phase beziehungsweise eine amorphe Phase in einem Verhältnisbereich von 0 - 15 : 85 - 100, wenn sie bei Umgebungsraumtemperatur fest ist.

**10.** Der Gegenstand gemäß Anspruch 6, wobei der besagte Gegenstand eine Laminatstruktur ist, die eine Filmschicht aufweist, die aus einer Extrusion der besagten thermoplastischen Zusammensetzung hergestellt ist.

**11.** Der Gegenstand gemäß Anspruch 6, wobei der Gegenstand a) ein Film, Faser, Fasergewebe, Saugkissen, Windel, Erwachseneninkontinenz- oder weibliches Hygieneprodukt, Schutzgewebe, Gesichtsmaske, Abdecktuch, Wischtuch, Kleidungsstück und Verpackungsgegenstand ist; oder b) ein Endotrachealtubus, Katheterschlauch, ein Heizbalg oder Ballon ist.

**12.** Ein Verfahren zum Weichmachen eines eine kristalline Phase beinhaltenden Polymers, das Verfahren weist auf: Bereitstellen eines thermoplastischen Ausgangspolymers mit 14 bis 25 % Kristallinität und eines Weichmachmittels mit einer Xanthen-basierten Molekularstruktur, vorhanden in einer Menge von bis zu 5000 - 6000 ppm der gesamten Zusammensetzung, in einer Mischung; Mischen des besagten thermoplastischen Polymers und des besagten Weichmachmittels in einem geschmolzenen oder flüssigen Zustand zwischen einem Temperaturbereich von 140-300 °C; und gleichmäßig Dispergieren des besagten Weichmachmittels durch die besagte geschmolzene Mischung; und Verfestigen der besagten geschmolzenen Mischung, so dass die besagte Xanthen-basierte Molekularstruktur sich in eine amorphe Phase umwandelt, wobei das thermoplastische Ausgangspolymer eine Polyalkylcarbonsäure ist und wobei der Kristallinitätsanteil der Polyalkylcarbonsäure aus dem Kristallinitätsindex berechnet wird:

$$X\% = \frac{I_c}{I_t} = \frac{\int i_c\, k^2 dk}{\int i_t k^2 dk} \quad (1)$$

wobei $I_c$ die XRD-Intensität gestreut von dem kristallinen Teil des Materials ist, $I_t$ die gesamte XRD-Intensität ist und k der Streuvektor mit einer Magnitude k=4πsinθ/λ ist (λ ist die Wellenlänge der Röntgenstrahlung und 6 ist der Streuwinkel).

**13.** Das Verfahren gemäß Anspruch 12, wobei weiter aufweist Extrudieren oder Formen der besagten geschmolzenen Mischungen in einen Festkörper, wenn sie etwa bei Umgebungsraumtemperatur sind.

**14.** Das Verfahren gemäß Anspruch 12, wobei, wenn verfestigt, der resultierende Festkörper einen Kristallinitätsgrad aufweist, der zumindest 40 % bis zu 500 % weniger als ein thermoplastisches Originalpolymer ohne das besagte Weichmachmittel ist.

**15.** Das Verfahren gemäß Anspruch 12, wobei die besagte Mischung auf eine Temperatur zwischen 170 °C und 280 °C erhitzt wird.

## Revendications

**1.** Composition de polymère thermoplastique comprenant : un polymère semi-cristallin formant base de départ avec une teneur cristalline minimale de 14 % à 25 % en poids du polymère, et un composé plastifiant ayant une structure moléculaire à base de xanthène dans une quantité allant jusqu'à 6 000 ppm, de préférence de 3 000 ppm à 5 000 ppm, dispersés en son sein, et ledit mélange de composition mélangée ayant une phase cristalline et une phase amorphe dans une plage de rapports de 0-15:85-100, respectivement quand solide à température ambiante, dans laquelle ledit composé plastifiant est dispersé à l'intérieur de ladite phase amorphe ; dans laquelle ledit polymère semi-cristallin est un acide polyalkylcarboxylique ; et dans laquelle la teneur cristalline de l'acide polyalkylcarboxylique est calculée à partir de l'indice de cristallinité :

$$X\% = \frac{I_c}{I_t} = \frac{\int i_c k^2 dk}{\int i_t k^2 dk} \quad (1)$$

où $I_c$ est l'intensité XRD dispersée depuis la partie cristalline de la matière, $I_c$ est l'intensité XRD totale et k est le vecteur de dispersion avec une amplitude k = 4πsinθ/λ (λ est la longueur d'onde du rayonnement par rayons X et 6 est l'angle de dispersion).

**2.** Composition de polymère thermoplastique selon la revendication 1, dans laquelle ladite composition mélangée donne ou présente un niveau de cristallinité d'au moins 40 à 99 % inférieure à un polymère thermoplastique témoin initial sans ledit composé plastifiant ayant une structure moléculaire à base de xanthène.

**3.** Composition de polymère thermoplastique selon la revendication 1, dans laquelle ledit composé plastifiant a au moins un groupe R, où R est de l'hydrogène ou un halogène.

**4.** Composition thermoplastique selon la revendication 1, dans laquelle ladite structure moléculaire à base de xanthène est halogénée ou mixte-halogénée.

**5.** Composition de polymère thermoplastique selon la revendication 1, dans laquelle ladite phase cristalline est réduite d'une quantité de 40 % à 500 % par rapport au pourcentage de phase cristalline d'une composition identique absent dudit composé plastifiant ayant la structure moléculaire à base de xanthène ; ou dans laquelle ladite composition peut augmenter la dureté par rapport à un polymère thermoplastique sous-jacent n'ayant pas ledit composé plastifiant d'au moins 40 % à 60 % ; ou

dans laquelle ladite composition augmente en ce qui concerne la tolérance d'allongement par étirement par rapport à un polymère thermoplastique sous-jacent n'ayant pas de composé plastifiant d'au moins 40 % à 50 % ; ou dans laquelle ladite composition présente une augmentation de la déformation à la rupture dudit polymère par rapport à un polymère thermoplastique sous-jacent n'ayant pas de composé plastifiant d'au moins 40 % ; ou

dans laquelle ladite composition présente une biodégradation et une cinétique de compostage plus rapides en comparaison au polymère témoin sans le xanthène ou l'additif à base de xanthènes et est encore plus dure que le polymère témoin sans les xanthènes ou l'additif à base de xanthène ; et dans laquelle ladite composition est sensiblement amorphe et plus dure que le polymère témoin sans les xanthènes ou l'additif à base de xanthène ;

dans laquelle la dureté, l'allongement par étirement et la déformation à la rupture sont mesurés sur un film ayant une épaisseur de 0,25 mm, une longueur de 20 mm et une largeur de 3 mm, ledit film étant moulé par compression à 225 °C et refroidi jusqu'à température ambiante à une vitesse de 10 °C/min, et testé à 40 °C avec la force appliquée augmentée à un taux de 2 N/min jusqu'à la rupture.

**6.** Article de fabrication comprenant un polymère thermoplastique, le polymère thermoplastique ayant une matrice de polymère semi-cristallin incorporant un plastifiant comprenant une molécule de xanthène ou un composé ayant une structure moléculaire à base de xanthène, ledit polymère semi-cristallin contient une teneur cristalline de 0 % à 15 % de phase cristalline, 100 % à 85 % d'état amorphe, ladite molécule de xanthène ou le composé ayant une structure moléculaire à base de xanthène est présent dans ladite matrice de polymère dans une quantité de 3 000 ppm jusqu'à 5 000 ppm ; dans lequel ledit polymère semi-cristallin est un acide polyalkylcarboxylique ; et dans lequel la

teneur cristalline de l'acide polyalkylcarboxylique est calculée à partir de l'indice de cristallinité :

$$X\% = \frac{I_c}{I_t} = \frac{\int i_c k^2 dk}{\int i_t k^2 dk} \quad (1)$$

où $I_c$ est l'intensité XRD dispersée depuis la partie cristalline de la matière, $I_c$ est l'intensité XRD totale et k est le vecteur de dispersion ayant une amplitude k = 4πsinθ/λ, (λ est la longueur d'onde du rayonnement par rayons X et 6 est l'angle de dispersion).

**7.** Article selon la revendication 6, dans lequel ladite molécule de xanthènes ou composé ayant une structure moléculaire à base de xanthène est sélectionné à partir d'un composé à base de xanthènes inchangé, halogéné, ou mixte-halogéné.

**8.** Composition de polymère thermoplastique selon la revendication 1 ou l'article selon la revendication 6, dans lequel ledit polymère semi-cristallin est de l'acide polylactique.

**9.** Article selon la revendication 6, dans lequel ledit article est une fibre ou un filament, ou un voile de fibres, de préférence dans lequel ladite fibre fait partie d'un tissu tissé ou ledit voile de fibres forme un tissu non tissé, fait à partir d'une extrusion de ladite composition thermoplastique comprenant un polymère semi-cristallin de départ ayant une teneur cristalline minimale de 14 % en poids du polymère, et un composé ayant une structure moléculaire à base de xanthène dans une quantité allant jusqu'à 5 000 à 6 000 ppm dispersé en son sein, et ladite composition finale mélangée ayant une phase cristalline et une phase amorphe dans une plage de rapports de 0-15:85-100, respectivement quand solide à température ambiante.

**10.** Article selon la revendication 6, dans lequel ledit article est une structure de stratifié comprenant une couche de film faite à partir d'une extrusion de ladite composition thermoplastique.

**11.** Article selon la revendication 6, dans lequel l'article est : a) un film, une fibre, un voile de fibres, un coussinet absorbant, une couche, un produit lié à l'incontinence adulte ou un produit d'hygiène féminine, un tissu protecteur, un masque pour le visage, un champ médical, un essuyeur, un vêtement et un article d'emballage ; ou b) un tube endotrachéal, un tubage de cathéter, une vessie ou un ballon.

**12.** Procédé de plastification d'un polymère contenant une phase cristalline, le procédé comprend :

la fourniture dans un mélange d'un polymère thermoplastique de départ ayant une cristallinité de 14 à 25 % et un agent plastifiant ayant une structure moléculaire à base de xanthène présente dans une quantité allant jusqu'à 5 000 à 6 000 ppm de composition totale ; le mélange dudit polymère thermoplastique et dudit agent plastifiant dans un état fondu ou liquide entre une plage de températures de 140 à 300 °C ; et la dispersion uniforme dudit agent plastifiant partout dans ledit mélange fondu ; et la solidification dudit mélange fondu de sorte que ladite structure moléculaire à base de xanthène migre dans une phase amorphe ; dans lequel le polymère thermoplastique de départ est un acide polyalkylcarboxylique ; et dans lequel la teneur cristalline de l'acide polyalkylcarboxylique est calculée à partir de l'indice de cristallinité :

$$X\% = \frac{I_c}{I_t} = \frac{\int i_c k^2 dk}{\int i_t k^2 dk} \quad (1)$$

où $I_c$ est l'intensité XRD dispersée depuis la partie cristalline de la matière, $I_c$ est l'intensité XRD totale et k est le vecteur de dispersion avec une amplitude k = 4πsinθ/λ (λ est la longueur d'onde du rayonnement par rayons X et 6 est l'angle de dispersion).

**13.** Procédé selon la revendication 12, dans lequel il comprend en outre l'extrusion ou la formation desdits mélanges fondus en un solide quand on est à peu près à température ambiante.

**14.** Procédé selon la revendication 12, dans lequel, quand il est solidifié, le solide résultant présente un niveau de

cristallinité qui est d'au moins 40 % jusqu'à 500 % inférieur au polymère thermoplastique original sans ledit agent plastifiant.

**15.** Procédé selon la revendication 12, dans lequel ledit mélange est chauffé jusqu'à une température comprise entre 170 °C et 280 °C.

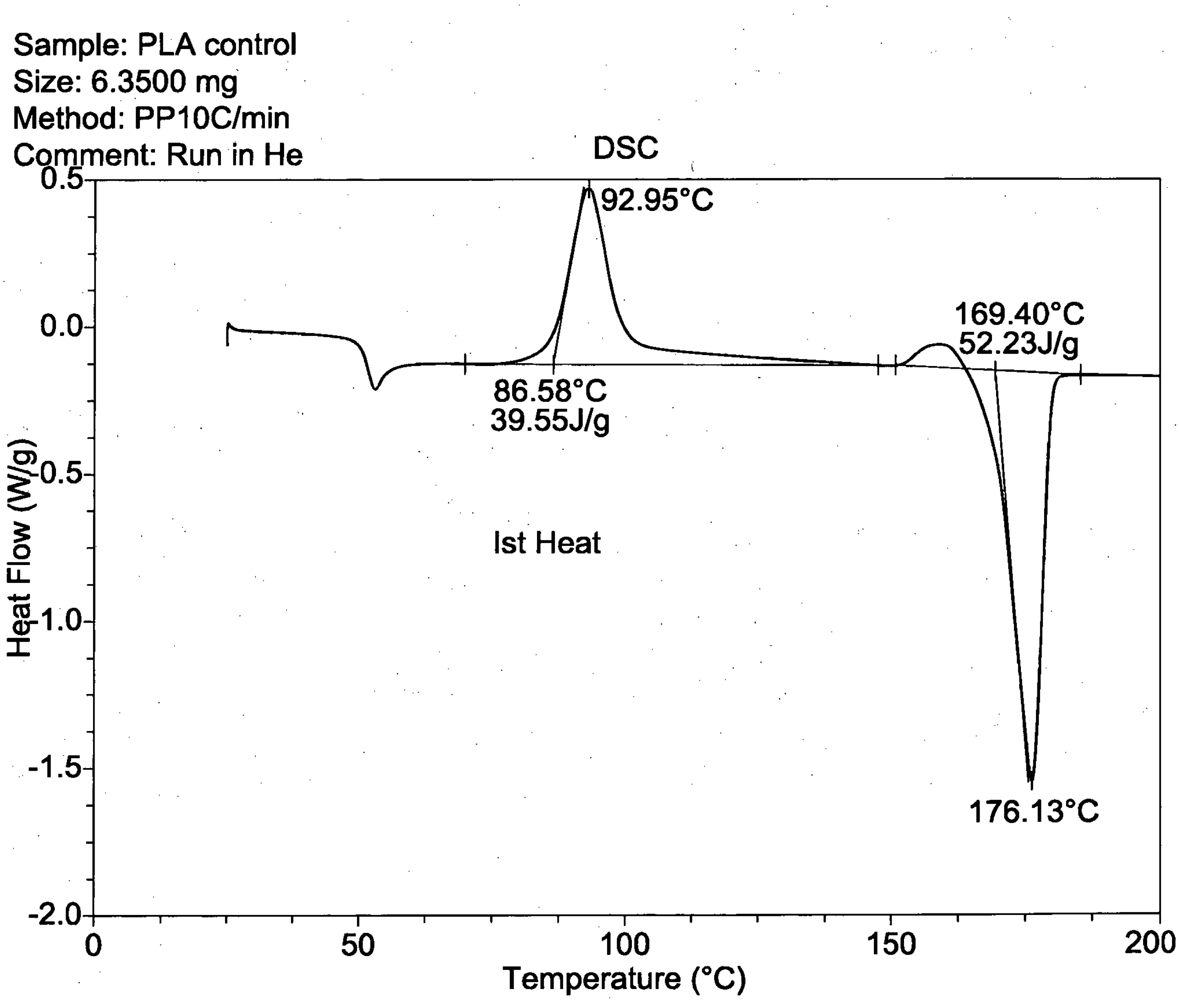


DSC curve of Control Sample (PLA control – solution cast); first heat.

FIG. 1

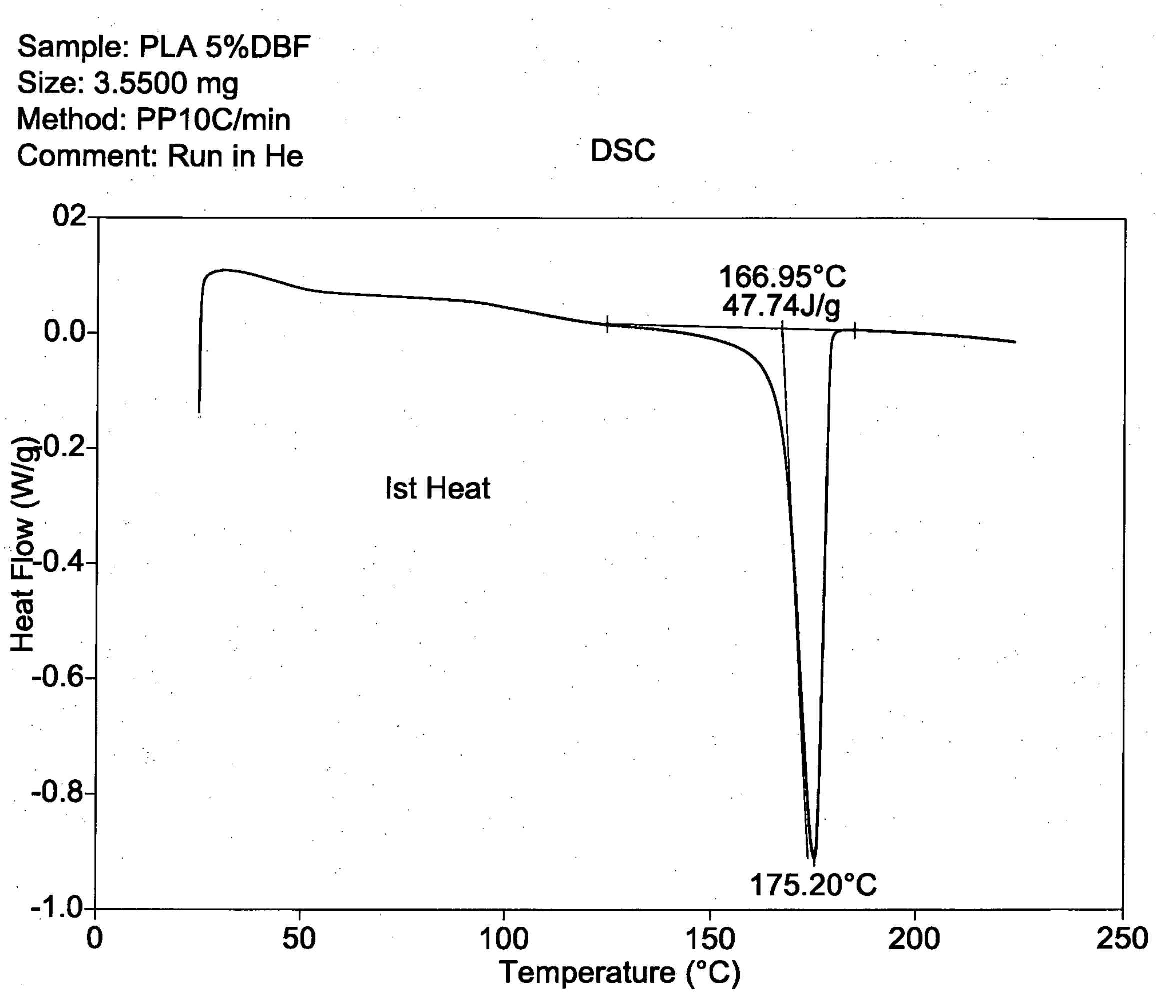


DSC curve of Example #1 (PLA+5000ppm DBF – solution cast); first heat.

FIG. 2

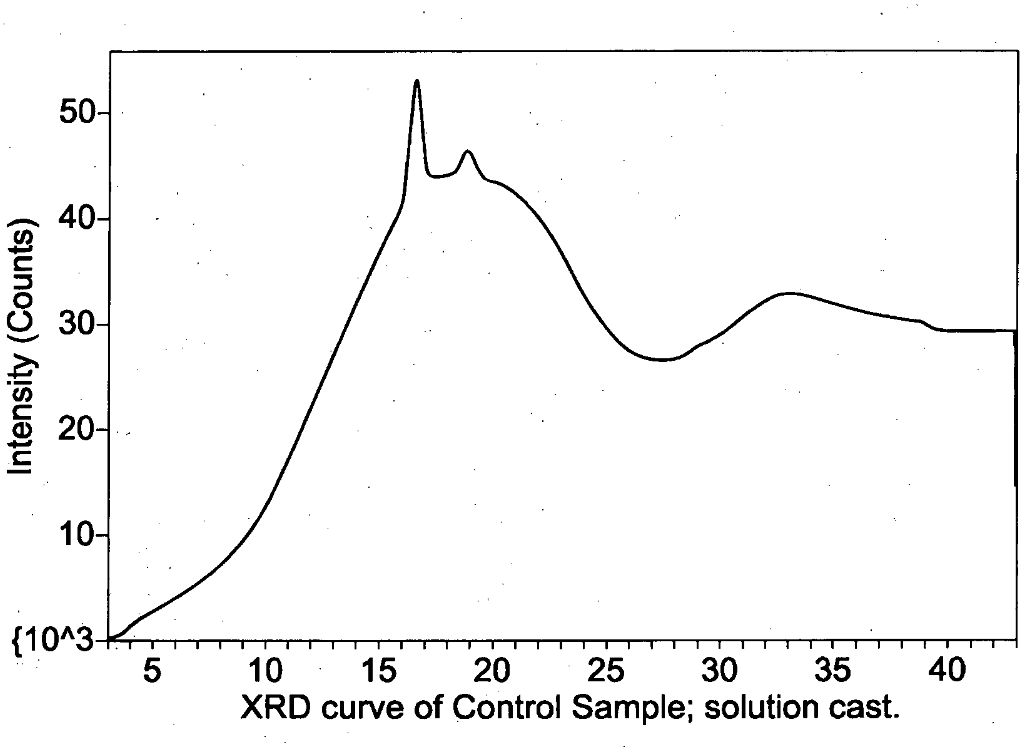
Intensity (Counts)
50
40
30
20
10
{10^3
5
10
15
20
25
30
35
40
XRD curve of Control Sample; solution cast.

FIG. 3

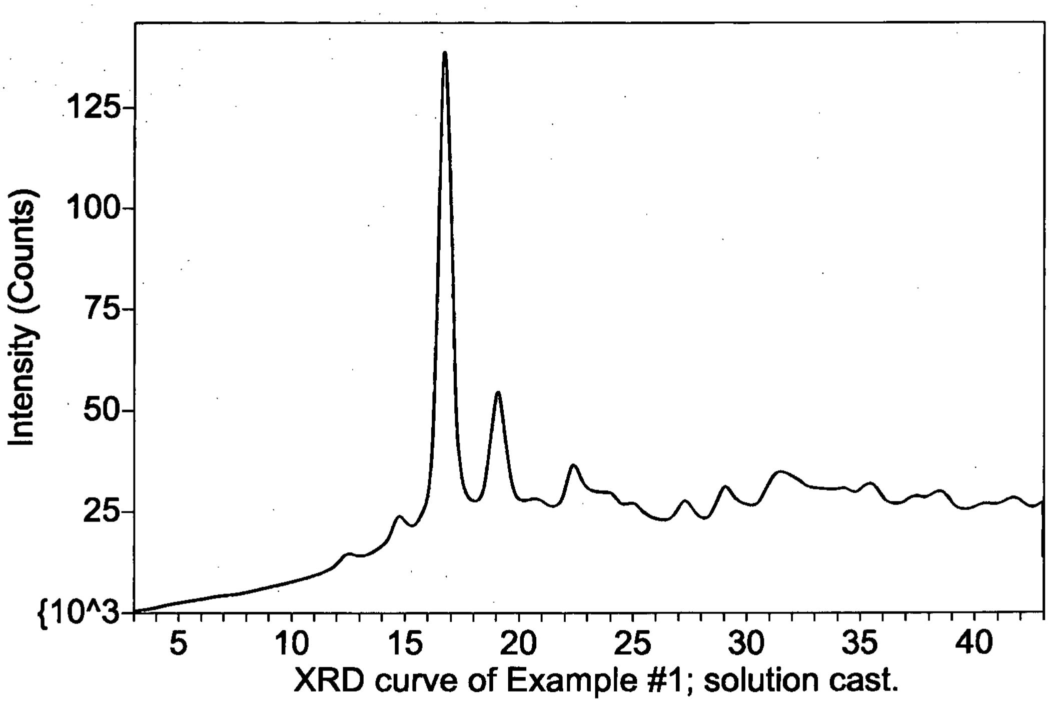
Intensity (Counts)
125
100
75
50
25
{10^3
5
10
15
20
25
30
35
40
XRD curve of Example #1; solution cast.

FIG. 4

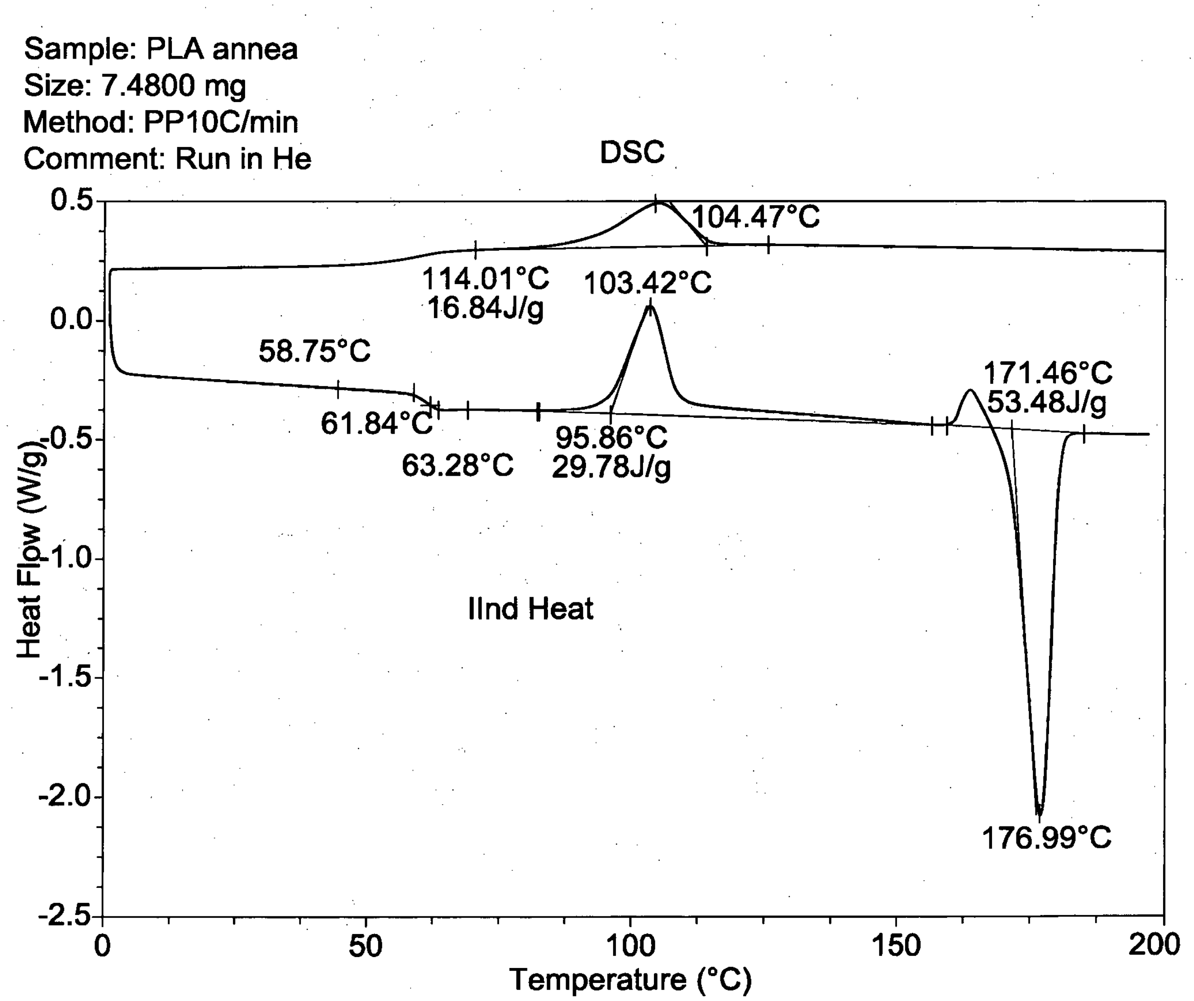


DSC curve of Control Sample (PLA control); cooling and IInd Heat.

FIG. 5

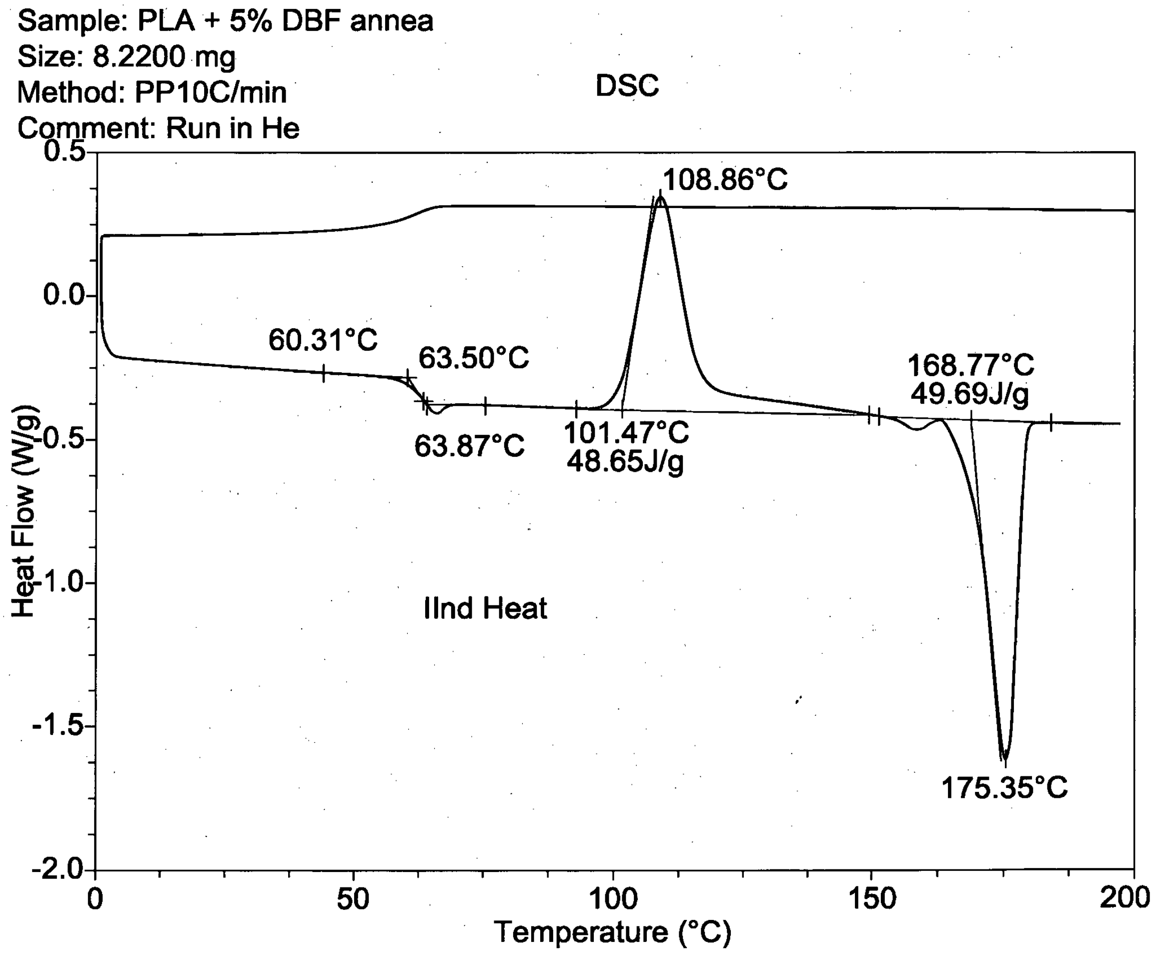


DSC curve of Example #1 (PLA+5000ppm DBF); cooling and II[nd] Heat.

FIG. 6

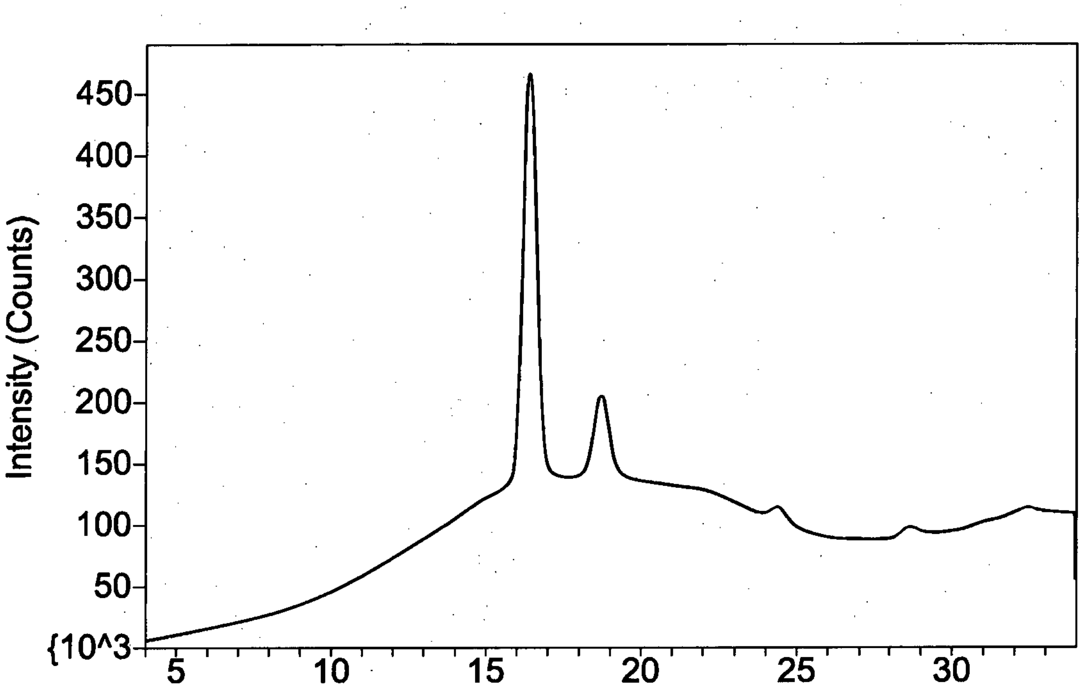

XRD curve of Control Sample after 10° C/min cooling.

FIG. 7

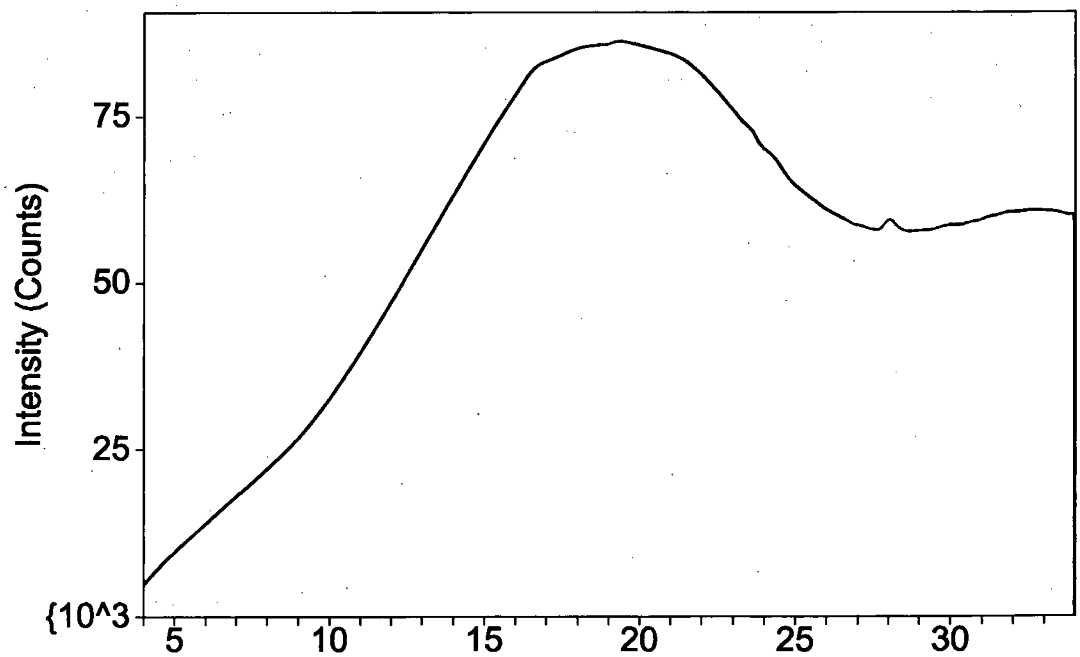

XRD curve of Example #1 after 10° C/min cooling.

FIG. 8

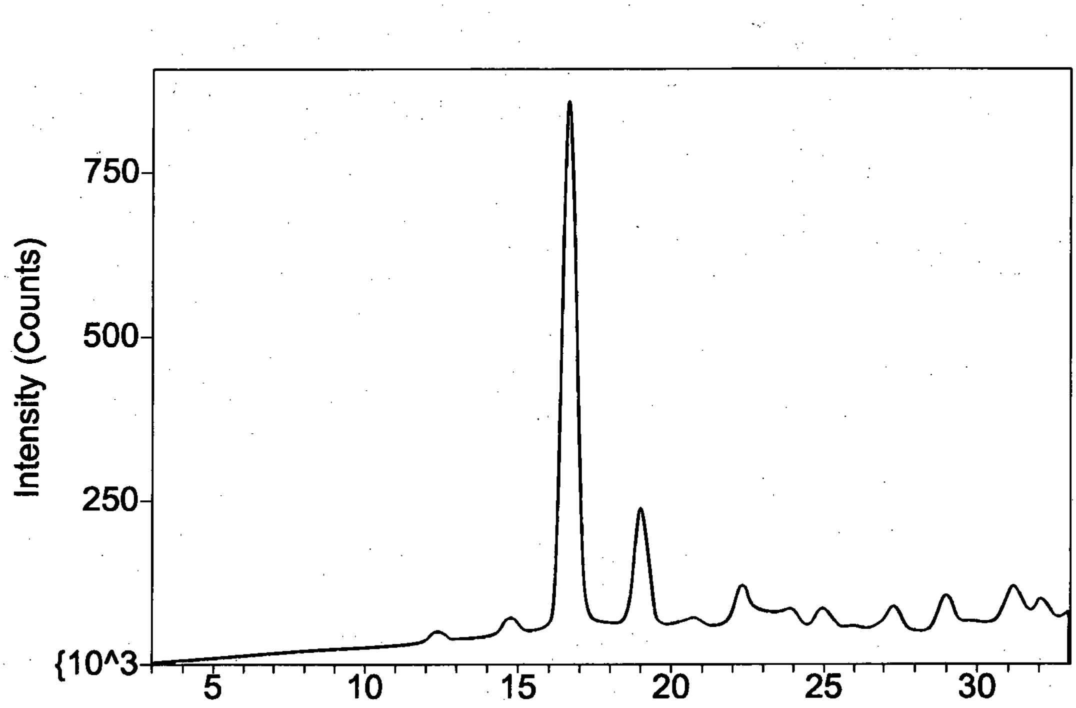


XRD curve of Control Sample after slow (oven) cooling.

FIG. 9

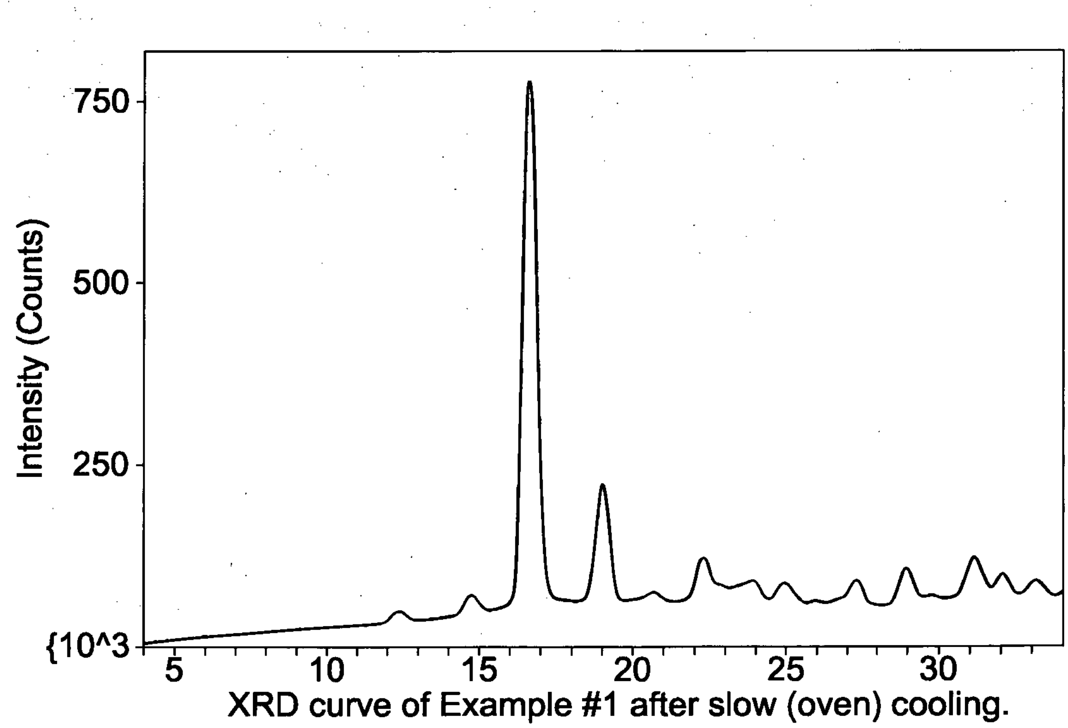


XRD curve of Example #1 after slow (oven) cooling.

FIG. 10

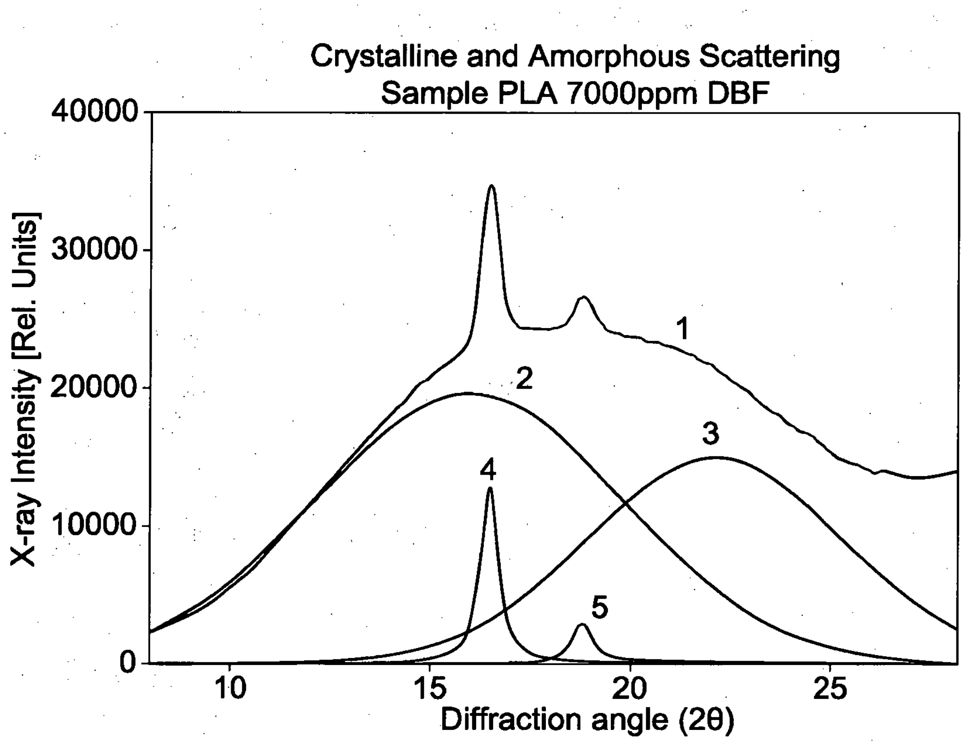


XRD experimental curve of Example #7 after cooling with 10° C/min (curve #1). Curves #2 and #3 are the two amorphous halos and curves #4 and #5 are the two crystalline peaks.

FIG. 11

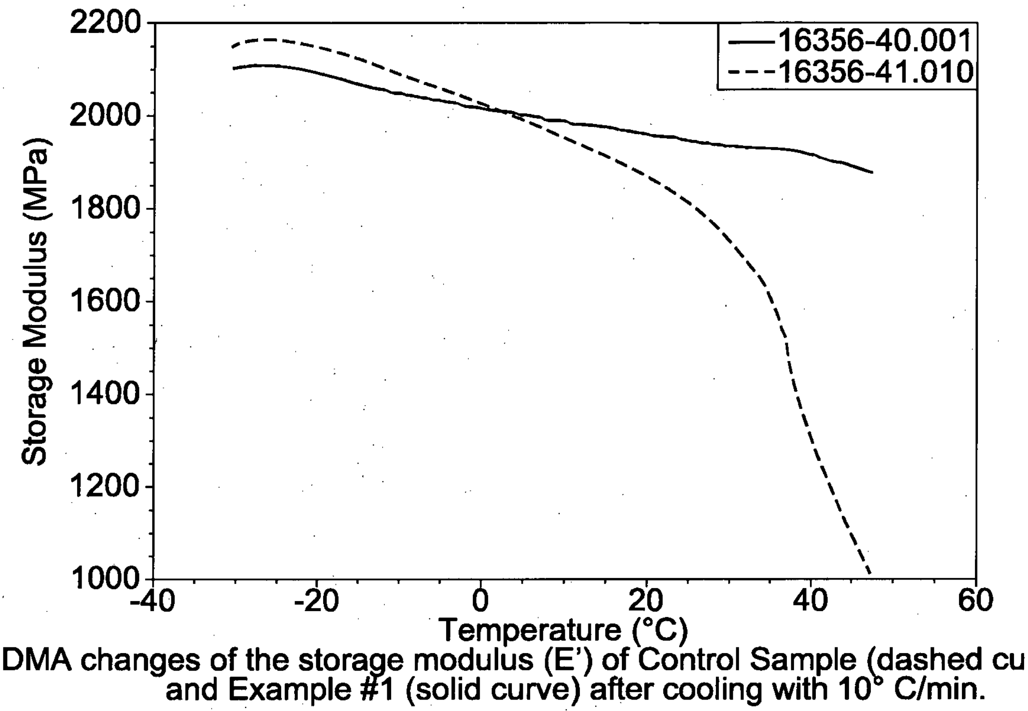


DMA changes of the storage modulus (E') of Control Sample (dashed curve) and and Example #1 (solid curve) after cooling with 10° C/min.

FIG. 12

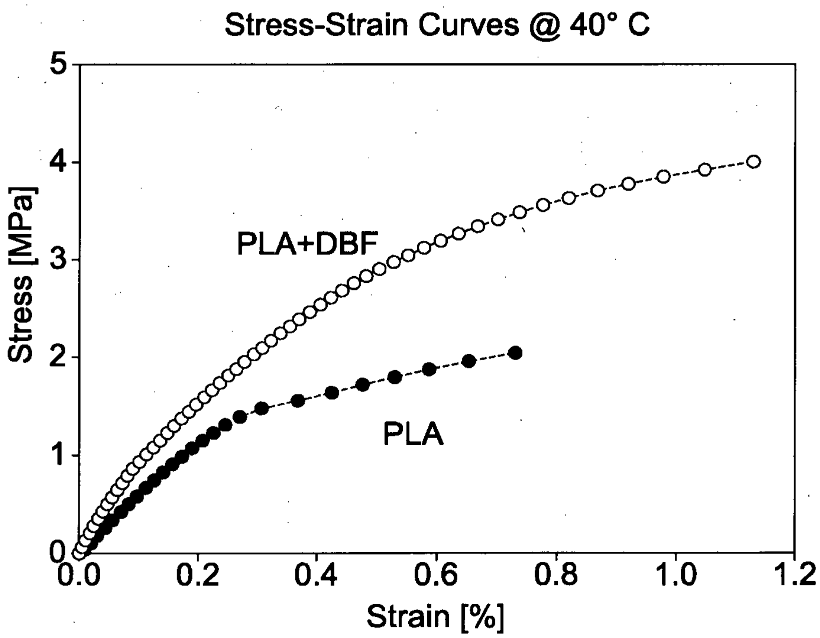


Stress-strain curves @ 40° C of Control Sample (PLA and 10° C/min cooling; solid circles) and Example#1 (PLA+5000ppm DBF and 10° C/min cooling; open circles).

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 974369 A
- US 11974393 B

### Non-patent literature cited in the description

- **HIDETO TSUJI ; YOSHITO IKADA.** *J. Appl . Polym. Sci.,* 1997, vol. 63, 855-863
- **LILLIE, H. J.** CONN's BIOLOGICAL STAINS. Williams & Wilkins, 1977, 326